(19) Europäisches Patentamt
European Patent Office
Office européen des brevets

(11) **EP 4 019 006 A1**

(12) **EUROPEAN PATENT APPLICATION**

(43) Date of publication:
**29.06.2022   Bulletin 2022/26**

(21) Application number: **21217405.6**

(22) Date of filing: **23.12.2021**

(51) International Patent Classification (IPC):
***A61K 9/127*** (2006.01)

(52) Cooperative Patent Classification (CPC):
**A61K 9/1271; A61K 9/1272**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:   **28.12.2020   US 202063131134 P**

(71) Applicant: **Industrial Technology Research Institute**
**Chutung, Hsinchu 310401 (TW)**

(72) Inventors:
• **Yu, Neng-Chang**
  **Kaohsiung City (TW)**
• **Cheng, Felice**
  **Hsinchu County (TW)**

• **Liu, Chih-Peng**
  **Hsinchu City (TW)**
• **Wu, Ming-Hsi**
  **Taipei City (TW)**
• **Chen, Shih-Ta**
  **New Taipei City (TW)**
• **Tu, Chia-Mu**
  **Taipei City (TW)**
• **Chang, Li-Wen**
  **New Taipei City (TW)**
• **Li, Jheng-Sian**
  **Taipei City (TW)**
• **She, Meng-Ping**
  **Hsinchu County (TW)**
• **Wang, Hsiang-Ching**
  **Hsinchu City (TW)**

(74) Representative: **Fuchs Patentanwälte Partnerschaft mbB**
**Westhafenplatz 1**
**60327 Frankfurt am Main (DE)**

(54) **IMMUNOSTIMULATORY LIPOPLEX, PHARMACEUTICAL COMPOSITION INCLUDING IMMUNOSTIMULATORY LIPOPLEX, AND USES THEREOF**

(57)    An immunostimulatory lipoplex is provided. The immunostimulatory lipoplex includes a liposome and at least one immunostimulatory nucleic acid drug, and the immunostimulatory nucleic acid drug is complexed with the liposome. The liposome includes 40 to 85 mol% of cationic lipid, 10 to 50 mol% of cholesterol, and 0.001 to 20 mol% of modified polyethylene glycol lipid. A pharmaceutical composition including the immunostimulatory lipoplex is also provided.

FIG. 1

**SEQUENCE LISTING**

```
<110>   Industrial Technology Research Institute

<120>   Immunostimulatory lipoplex, pharmaceutical composition including
        immunostimulatory lipoplex, and uses thereof

<130>   9044B-A28028

<160>   2

<170>   PatentIn version 3.5

<210>   1
<211>   24
<212>   DNA
<213>   Artificial Sequence

<220>
<223>   CpG-ODN


<220>
<221>   misc_difference
<222>   (1)..(23)
<223>   phosphorothioate bond

<400>   1
tcgtcgtttt gtcgttttgt cgtt                                      24


<210>   2
<211>   30
<212>   DNA
<213>   Artificial Sequence

<220>
<223>   CpG-ODN


<220>
<221>   misc_difference
<222>   (1)..(29)
<223>   phosphorothioate bond

<400>   2
tcgaacgttc gaacgttcga acgttcgaat                                30
```

**Description**

**CROSS REFERENCE TO RELATED APPLICATIONS**

This application claims the benefit of U.S. Provisional Application No. 63/131,134, filed December 28, 2020, the entirety of which is incorporated by reference herein.

**BACKGROUND**

**Technical Field**

The present disclosure relates to an immunostimulatory lipoplex and a pharmaceutical composition including the immunostimulatory lipoplex, and in particular it relates to an immunostimulatory lipoplex that can be effectively used in cancer treatment and a pharmaceutical composition thereof.

**Description of the Related Art**

Immunotherapy treats or prevents diseases by strengthening the human body's own immune system or imparting additional immunity. In recent years, immunotherapy has gradually been widely used in the treatment of cancer. There are various ways of performing immunotherapy, and immune checkpoint blockade (ICB) is considered to be an effective way to improve the effects of clinical cancer treatment. For example, an immune checkpoint inhibitor (ICI) can be used in the treatment. However, the efficacy of immune checkpoint inhibitors as monotherapy for the treatment of some cancers (e.g., colorectal cancer, triple-negative breast cancer, lung cancer, liver cancer, kidney cancer, etc.) is not ideal.

Furthermore, the safe mode of administration of several known immune checkpoint inhibitors (e.g., TLR9 activators) is intratumoral injection or subcutaneous injection. This limits the applicable indications of immune checkpoint inhibitors, for example, making them only applicable to the treatment of superficial tumors.

Therefore, the development of a delivery vehicle that can improve the therapeutic effect of immune checkpoint inhibitors (by increasing the treatment response rate and tissue exposure dose, for example) or increase its applicable indications is still one of the research goals in the pharmaceutical industry.

**SUMMARY**

In accordance with some embodiments of the present disclosure, an immunostimulatory lipoplex is provided. The immunostimulatory lipoplex includes a liposome and at least one immunostimulatory nucleic acid drug, and the immunostimulatory nucleic acid drug is complexed with the liposome. The liposome includes 40 to 85 mol% of cationic lipid, 10 to 50 mol% of cholesterol, and 0.001 to 20 mol% of modified polyethylene glycol (PEG) lipid. A pharmaceutical composition including the aforementioned immunostimulatory lipoplex is also provided in the present disclosure.

In accordance with some embodiments of the present disclosure, a use of the aforementioned immunostimulatory lipoplex is provided, which is used for the manufacture of a medicine for treating cancer.

In accordance with some embodiments of the present disclosure, a pharmaceutical composition is provided. The pharmaceutical composition includes the aforementioned immunostimulatory lipoplex.

In accordance with some embodiments of the present disclosure, a use of the aforementioned pharmaceutical composition for manufacturing a medicine for treating cancer is provided.

In order to make the features or advantages of the present disclosure clear and easy to understand, a detailed description is given in the following embodiments with reference to the accompanying drawings.

**BRIEF DESCRIPTION OF THE DRAWINGS**

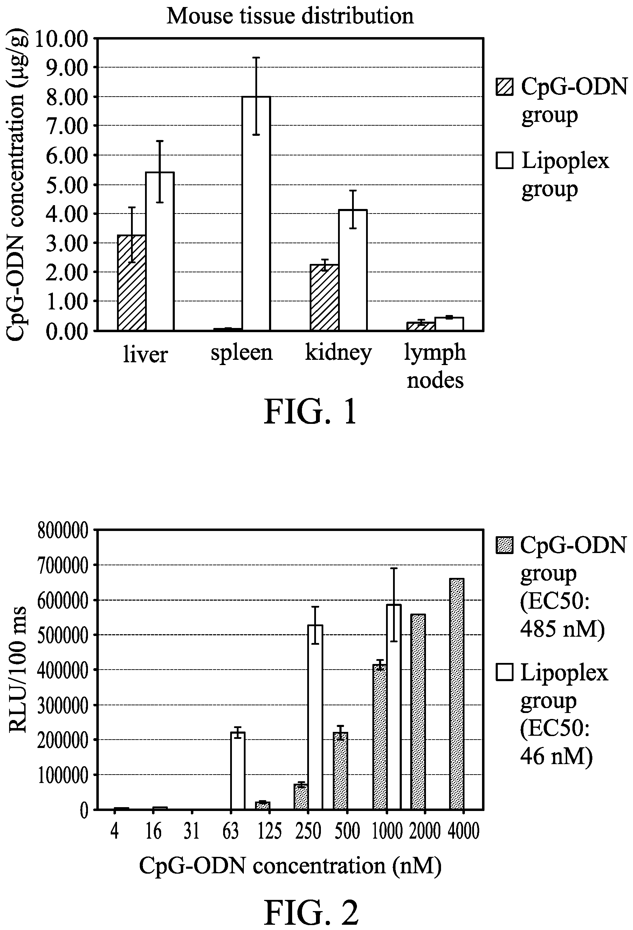

FIG. 1 shows the measurement results of the concentration of CpG oligodeoxynucleotides in mouse tissues after injection of CpG oligodeoxynucleotides and immunostimulatory lipoplexes in an embodiment of the present disclosure. CpG-ODN group: CpG-7909 oligodeoxynucleotide was intravenously injected into mice at a single dose of 50 μg/mouse and 100 μL/mouse; Lipoplex group: lipoplex of formula F12 complexed with radiolabeled CpG-7909 was intravenously injected into mice at a single dose of 50 μg/mouse and 100 μL/mouse.

FIG. 2 shows the relative luminescence value (relative light unit, RLU) results of SEAP produced by HEK-Blue™ hTLR9 cell line expressing human TLR9 after treated with different concentrations of CpG oligodeoxynucleotides and immunostimulatory lipoplexes in an embodiment of the present disclosure. CpG-ODN group: cell group treated with CpG-7909 oligodeoxynucleotide, and $EC_{50}$ value was about 485 nM; Lipoplex group: cell group treated with lipoplex of formula C1, and $EC_{50}$ value was about 46 nM.

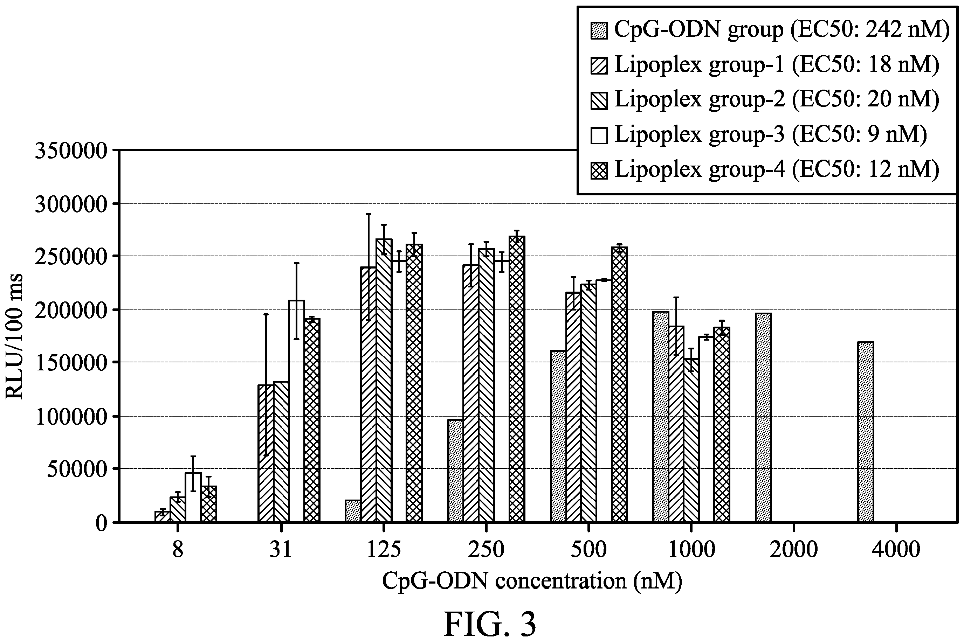

FIG. 3 shows the relative luminescence value (RLU) results of FLuc produced by HEK293-hTLR9/NF-$\kappa$B-luc cell line expressing human TLR9 after treated with different concentrations of CpG oligodeoxynucleotides and immunostimulatory lipoplexes in an embodiment of the present disclosure. CpG-ODN group: cell group treated with CpG-7909 oligodeoxynucleotide, and $EC_{50}$ value was about 242 nM; Lipoplex group-1: cell group treated with lipoplex of formula C1, and $EC_{50}$ value was about 18 nM; Lipoplex group-2: cell group treated with lipoplex of formula C3, and $EC_{50}$ value was about 20 nM; Lipoplex group-3: cell group treated with lipoplex of formula C2, and $EC_{50}$ value was about 9 nM; Lipoplex group-4: cell group treated with lipoplex of formula C6, and $EC_{50}$ value was about 12 nM.

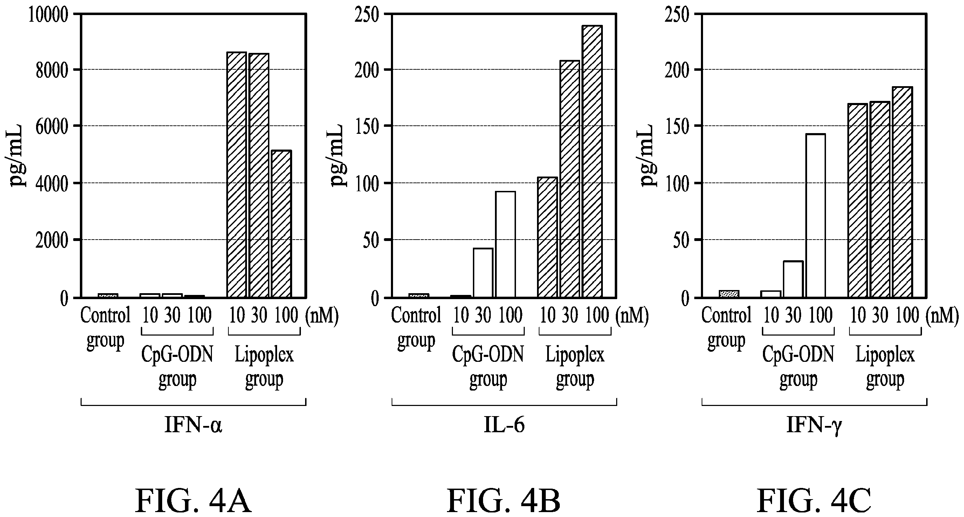

FIGs. 4A to 4C respectively show the concentration changes of IFN-$\alpha$, IL-6 and IFN-$\gamma$ produced by peripheral blood mononuclear cells (PBMC) after treated with different concentrations of CpG oligodeoxynucleotides and immunostimulatory lipoplexes in an embodiment of the present disclosure. Control group: 10 mM Tris buffer, pH 7.5; CpG-ODN group: PBMC group treated with CpG-7909 oligodeoxynucleotides; Lipoplex group: PBMC group treated with lipoplex of formula C1.

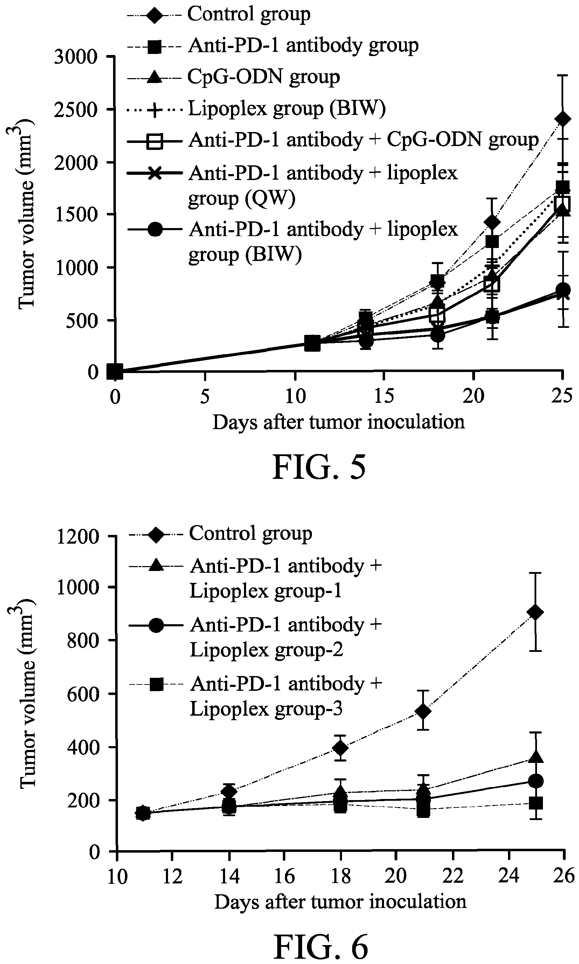

FIG. 5 shows the change of tumor volume in mice inoculated with CT26 tumor cell line after administration in an embodiment of the present disclosure. Control group: normal saline was intravenously injected twice a week; Anti-PD-1 antibody group: anti-mouse PD-1 antibody (250 $\mu$g/mouse) was intraperitoneally injected twice a week; CpG-ODN group: CpG-7909 (50 $\mu$g/mouse) was intravenously injected twice a week; Lipoplex group (BIW): lipoplex of formula C1 (50 $\mu$g/mouse) was intravenously injected twice a week; Anti-PD-1 antibody + CpG-ODN group: anti-PD-1 antibody (250 $\mu$g/mouse) was intraperitoneally injected twice a week and CpG-7909 (50 $\mu$g/mouse) was intravenously injected twice a week; Anti-PD-1 antibody + lipoplex group (QW): anti-PD-1 antibody (250 $\mu$g/mouse) was intraperitoneally injected twice a week and lipoplex of formula C1 (50 $\mu$g/mouse) was intravenously injected once a week; Anti-PD-1 antibody + lipoplex group (BIW): anti-PD-1 antibody (250 $\mu$g/mouse) was intraperitoneally injected twice a week and lipoplex of formula C1 (50 $\mu$g/mouse) was intravenously injected twice a week.

FIG. 6 shows the change of tumor volume in mice inoculated with CT26 tumor cell line after administration in an embodiment of the present disclosure. Control group: normal saline was intravenously injected twice a week; Anti-PD-1 antibody + lipoplex group-1: anti-PD-1 antibody (250 $\mu$g/mouse) was intraperitoneally injected twice a week and lipoplex of formula C10 (15 $\mu$g/mouse) was intravenously injected twice a week; Anti-PD-1 antibody + lipoplex group-2: anti-PD-1 antibody (250 $\mu$g/mouse) was intraperitoneally injected twice a week and lipoplex of formula C9 (15 $\mu$g/mouse) was intravenously injected twice a week; Anti-PD antibody + lipoplex group-3: anti-PD-1 antibody (250 $\mu$g/mouse) was intraperitoneally injected twice a week and lipoplex of formula C8 (15 $\mu$g/mouse) was intravenously injected twice a week.

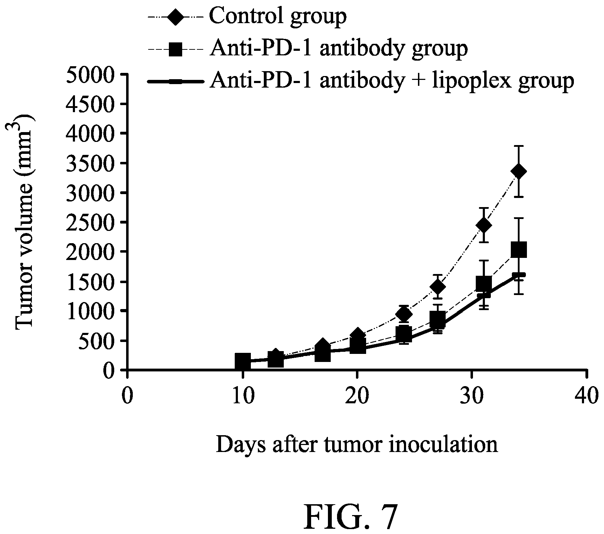

FIG. 7 shows the change of tumor volume in mice inoculated with CT26 tumor cell line after administration in a comparative example of the present disclosure. Control group: normal saline was intravenously injected twice a week; Anti-PD-1 antibody group: anti-PD-1 antibody (250 $\mu$g/mouse) was intraperitoneally injected twice a week; Anti-PD-1 antibody + lipoplex group: anti-PD-1 antibody (250 $\mu$g/mouse) was intraperitoneally injected twice a week and lipoplex of formula E2 (15 $\mu$g/mouse) was intravenously injected once a week.

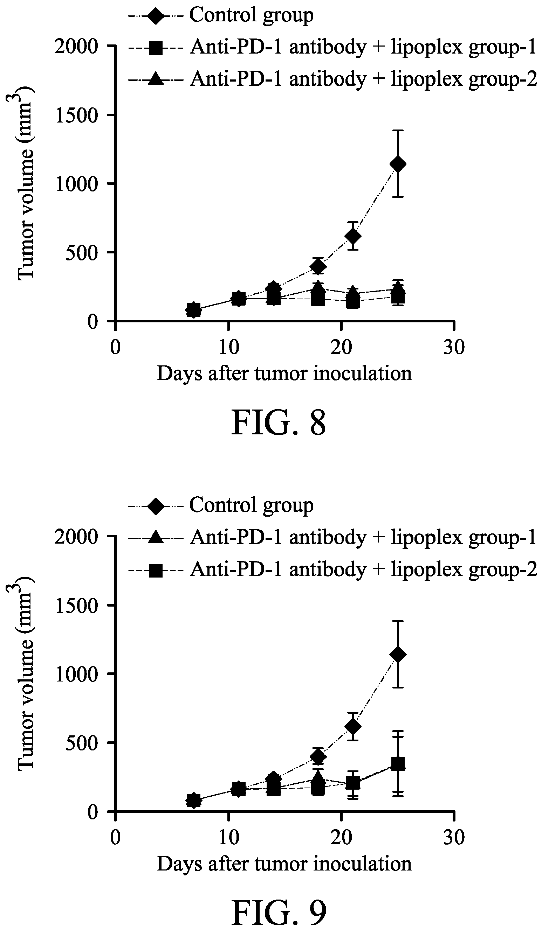

FIG. 8 shows the change of tumor volume in mice inoculated with CT26 tumor cell line after administration in an embodiment of the present disclosure. Control group: normal saline was intravenously injected twice a week; Anti-PD-1 antibody + lipoplex group-1: anti-PD-1 antibody (250 $\mu$g/mouse) was intraperitoneally injected twice a week and lipoplex of formula C8 (15 $\mu$g/mouse) was intravenously injected once a week; Anti-PD-1 antibody + lipoplex group-2: anti-PD-1 antibody (250 $\mu$g/mouse) was intraperitoneally injected twice a week and lipoplex of formula C14 (15 $\mu$g/mouse) was intravenously injected once a week.

FIG. 9 shows the change of tumor volume in mice inoculated with CT26 tumor cell line after administration in an embodiment of the present disclosure. Control group: normal saline was intravenously injected twice a week; Anti-PD-1 antibody + lipoplex group-1: anti-PD-1 antibody (250 $\mu$g/mouse) was intraperitoneally injected twice a week and lipoplex of formula C11 (15 $\mu$g/mouse) was intravenously injected once a week; Anti-PD-1 antibody + lipoplex group-2: anti-PD-1 antibody (250 $\mu$g/mouse) was intraperitoneally injected twice a week and lipoplex of formula C13 (15 $\mu$g/mouse) was intravenously injected once a week.

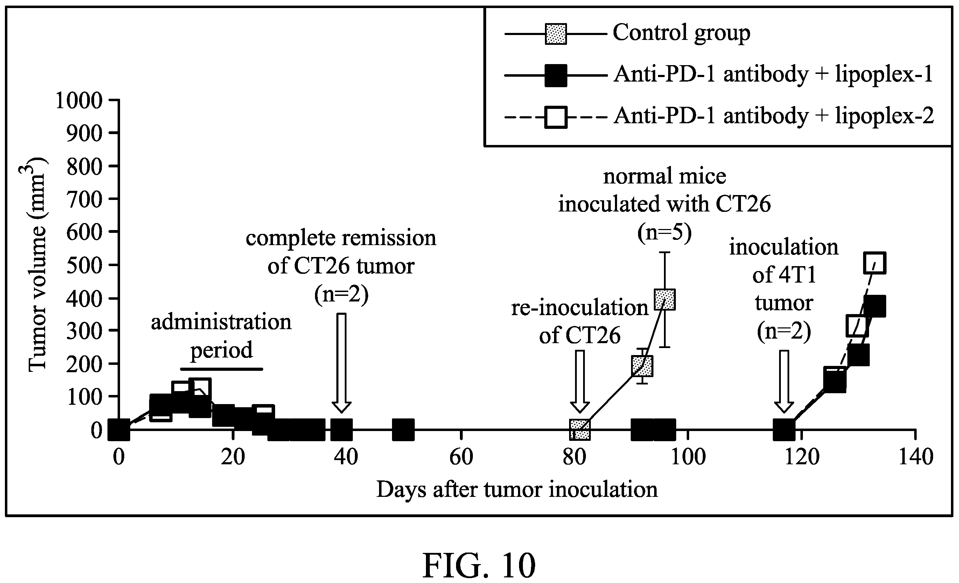

FIG. 10 shows the change of tumor volume in mice inoculated with CT26 tumor cell line after administration in an embodiment of the present disclosure. Anti-PD-1 antibody + lipoplex-1 and anti-PD-1 antibody + lipoplex-2: Two mice were each given intraperitoneal injection of anti-PD-1 antibody (250 $\mu$g/mouse) twice a week and intravenous injection of lipoplex of formula C1 (50 $\mu$g/mouse). The period of drug administration was 14 days (the eleventh day to the twenty-fifth day). Control group: CT26 cell line was re-inoculated into normal mice on the 81st day.

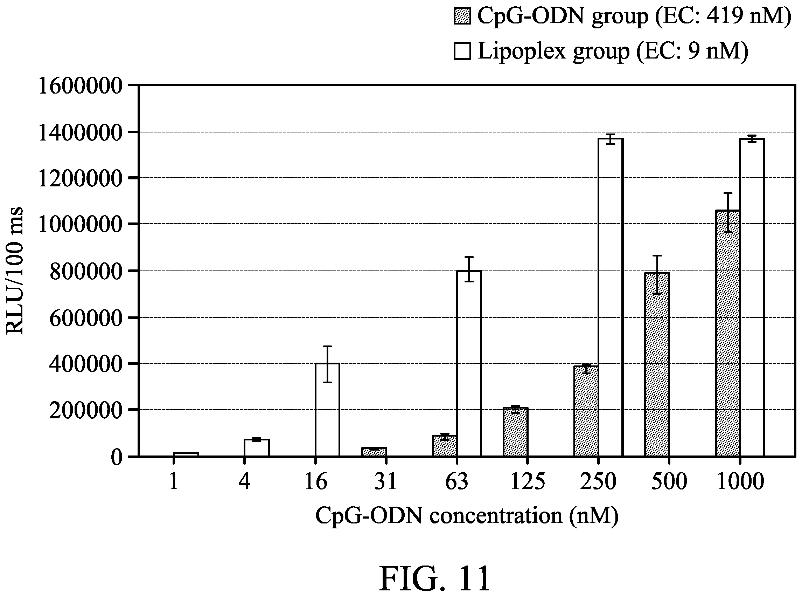

FIG. 11 shows the relative luminescence value (RLU) results of SEAP produced by HEK-Blue™ mTLR9 cell line expressing mouse TLR9 after treated with different concentrations of CpG oligodeoxynucleotides and immunostimulatory lipoplexes in an embodiment of the present disclosure. CpG-ODN group: cell group treated with CpG-30-

PS oligodeoxynucleotide, and $EC_{50}$ value was about 419 nM; Lipoplex group: cell group treated with lipoplex of formula C20, and $EC_{50}$ value was about 9 nM.

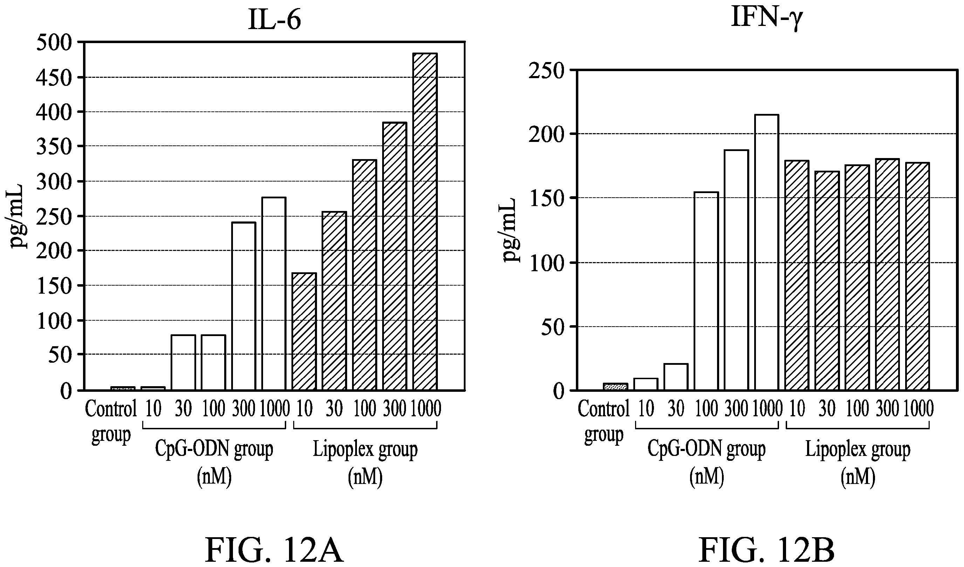

FIGs. 12A to 12B respectively show the concentration changes of IL-6 and IFN-$\gamma$ produced by peripheral blood mononuclear cells (PBMC) after treated with different concentrations of CpG oligodeoxynucleotides and immunostimulatory lipoplexes in an embodiment of the present disclosure. Control group: 10 mM Tris buffer, pH 7.5; CpG-ODN group: PBMC group treated with CpG-30-PS oligodeoxynucleotides; Lipoplex group: PBMC group treated with lipoplex of formula C20.

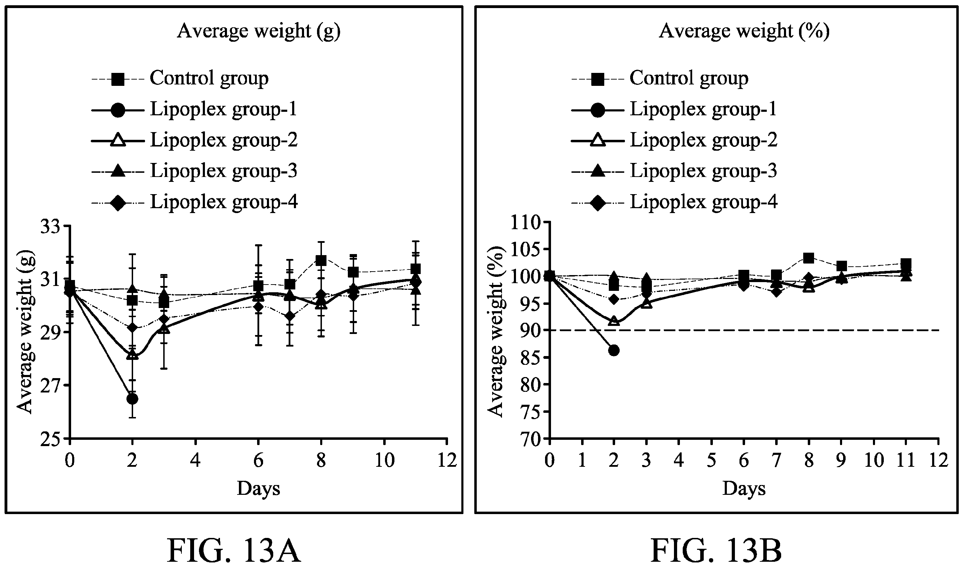

FIGs. 13A to 13B show changes in body weight of mice after administration in an embodiment of the present disclosure. Control group: normal saline was intravenously injected twice a week; Lipoplex group-1: lipoplex of formula C15 (50 $\mu$g/mouse) was intravenously injected once a week; Lipoplex group-2: lipoplex of formula C2 (50 $\mu$g/mouse) was intravenously injected once a week; Lipoplex group-3: lipoplex of formula C9 (15 $\mu$g/mouse) was intravenously injected once a week; Lipoplex group-4: lipoplex of formula C19 (5 $\mu$g/mouse) was intravenously injected once a week.

## DETAILED DESCRIPTION

The nucleic acid-drug complex of the present disclosure is described in detail in the following description. It should be understood that in the following detailed description, for purposes of explanation, numerous specific details and embodiments are set forth in order to provide a thorough understanding of the present disclosure. The specific elements and configurations described in the following detailed description are set forth in order to clearly describe the present disclosure. It will be apparent that the exemplary embodiments set forth herein are used merely for the purpose of illustration and not the limitation of the present disclosure.

In the following description, the terms "about", "approximately" and "substantially" typically mean +/- 5% of the stated value, or typically +/- 3% of the stated value, or typically +/- 2% of the stated value, or typically +/- 1% of the stated value or typically +/- 0.5% of the stated value. The stated value of the present disclosure is an approximate value. When there is no specific description, the stated value includes the meaning of "about", "approximately" and "substantially". In addition, the term "in a range from the first value to the second value" and "is from the first value to the second value" means that the range includes the first value, the second value, and other values in between.

Unless defined otherwise, all technical and scientific terms used herein have the same meaning as commonly understood by one of ordinary skill in the art to which this disclosure belongs. It should be appreciated that, in each case, the term, which is defined in a commonly used dictionary, should be interpreted as having a meaning that conforms to the relative skills of the present disclosure and the background or the context of the present disclosure, and should not be interpreted in an idealized or overly formal manner unless so defined.

The term "cationic lipid" refers to any of a variety of lipid species that have a net positive charge at physiological pH or have protonatable groups and are positively charged at pH below the pKa.

The terms "nucleic acid", "oligonucleotide", "polynucleotide" and "nucleic acid molecule" may be used interchangeably herein to refer to a polymer of nucleotides of any length, which may include a single-stranded DNA (ssDNA), a double-stranded DNA (dsDNA), a single-stranded RNA (ssRNA) and a double-stranded RNA (dsRNA). Nucleotides may be deoxyribonucleotides, ribonucleotides, or modified nucleotides. Nucleosides consist of purine (adenine (A) or guanine (G) or their derivatives) or pyrimidine (thymine (T), cytosine (C) or uracil (U) or their derivatives) bases and sugars bonds. The four nucleoside units (or bases) in DNA are called deoxyadenosine, deoxyguanosine, deoxythymidine and deoxycytidine. The four nucleoside units (or bases) in RNA are called adenosine, guanosine, uridine and cytidine. Nucleotides are phosphate esters of nucleosides. Non-limiting examples of nucleic acids include genes or gene fragments, exons, introns, messenger RNA (mRNA), transfer RNA, ribosomal RNA, ribozymes, cDNA, RNAi, siRNA, miRNA, recombinant polynucleotides, branched polynucleotides, plasmids, vectors, isolated DNA of any sequence, isolated RNA of any sequence, nucleic acid probes and primers, etc. Nucleic acids may include modified nucleotides, such as methylated nucleotides and nucleotide analogs.

The terms "CpG" and "CG" may be used interchangeably herein to refer to a cytosine and a guanine that are separated by a phosphodiester bond. In accordance with some embodiments of the present disclosure, the oligonucleotide may include one or more unmethylated CpG dinucleotides. In accordance with some embodiments of the present disclosure, the oligonucleotide may be an oligodeoxynucleotide (ODN).

The term " programmed death ligand-1", also known as "PD-L1", "cluster of differentiation 274 (CD274)" or "B7 homolog-1 (B7-H1)", refers to the protein encoded by CD274 gene in humans. Human PD-L1 is a 40 kDa type 1 transmembrane protein, whose main function is suppressing the immune system. PD-L1 binds to the receptor PD-1 on activated T cells, B cells and bone marrow cells to regulate activation or inhibition. PD-L1 also has a significant affinity for the costimulatory molecule CD80 (B7-1). The binding of PD-L1 to the receptor PD-1 on T cells can transmit a signal that can inhibit the production of IL-2 regulated by the T cell receptor and the activation of T cell proliferation. PD-L1 can be regarded as a checkpoint, and its increase in tumors facilitates the inhibition of the anti-tumor response regulated by T cells. In accordance with some embodiments of the present disclosure, PD-L1 may be PD-L1 derived from mammals, for example, may be PD-L1 derived from humans.

The term "cancer" refers to a physiological condition characterized by unregulated cell growth in a cell population in a mammal. The term "tumor" refers to any tissue mass produced by excessive cell growth or proliferation, which includes benign (non-cancerous) or malignant (cancerous) tumors, including precancerous lesions.

The term "immune response" includes the response from the innate immune system and the acquired immune system, which includes a cell-regulated immune response or a humoral immune response. The immune response includes T cell and B cell responses, as well as responses from other cells of the immune system, such as natural killer (NK) cells, monocytes, macrophages, etc.

Furthermore, the term "treatment" refers to a therapeutic measure that cures or alleviates the diagnosed pathological symptom or disease, reduces and/or stops the progression of the disease, and the preventive measures to prevent and/or alleviate the development of the target pathological symptom or disease. Therefore, the individuals in need of treatment may include those who already have a disease, those who are susceptible to a disease, and those who are to be prevented a disease. In accordance with the embodiments of the present disclosure, if a patient with cancer or tumor shows one or more conditions as follow, it means that the individual has been successfully treated: increased immune response, increased anti-tumor response, increased cytolytic activity of immune cells, and increased killing tumor cells by immune cells, decreased cancer cells or not existed at all; decreased tumor size; inhibited or absent cancer cell infiltration into surrounding organs; inhibited or absent tumor or cancer cell metastasis; inhibited or absent cancer cell growth; remission of one or more symptoms associated with a specific cancer; reduced morbidity and mortality; improved life quality; reduced tumorigenicity; or decreased number or occurrence frequency of cancer stem cells, etc.

In accordance with some embodiments of the present disclosure, an immunostimulatory lipoplex is provided, and it includes a liposome having a specific composition and an immunostimulatory nucleic acid drug that is complexed with the liposome. The immunostimulatory lipoplex can be administered using a systemic administration manner (for example, intravenous injection). The lipoplex provided in the embodiments of the present disclosure can improve the problems of poor stability and insufficient tissue exposure of immunostimulatory nucleic acid drugs (e.g., oligodeoxynucleotides). The specific immune activation of the drugs therefore can be improved, and the action time of a single dose of drugs can be prolonged, which reduces the systemic immune side effects caused by the drugs. Furthermore, in accordance with some embodiments of the present disclosure, the combined use of the immunostimulatory lipoplex and the current immune checkpoint inhibitor can exert a synergistic effect to further improve the efficacy of cancer immunotherapy.

In accordance with the embodiments of the present disclosure, the provided immunostimulatory lipoplex (lipoplex) includes a liposome and at least one immunostimulatory nucleic acid drug, and the immunostimulatory nucleic acid drug is complexed with the liposome. In addition, the liposome includes about 40 to 85 mole percent (mol%) of cationic lipid, about 10 to 50 mole percent of cholesterol, and about 0.001 to 20 mole percent of modified polyethylene glycol (PEG) lipid.

In accordance with some embodiments, the liposome includes about 50 to 80 mole percent of cationic lipid, about 15 to 35 mole percent of cholesterol, and about 0.01 to 15 mole percent of modified PEG lipid. In accordance with some embodiments, the liposome includes about 60 to 80 mole percent of cationic lipid, about 15 to 30 mole percent of cholesterol, and about 0.1 to 15 mole percent of modified PEG lipid.

In accordance with some embodiments, the liposome is nanoparticle assemblies formed by the composition including cationic lipid, cholesterol, and modified PEG lipid. As used herein, the term "nanoparticle" refers to a particle whose size is measured with a nanometer-scale. For example, a nanoparticle refers to a particle having a structure with a particle size of less than about 10000 nanometers (nm). In accordance with some embodiments, the particle size of the liposome is in a range from about 50 nm to about 200 nm, or from about 60 nm to about 170 nm, or from about 70 nm to about 165 nm, e.g., about 80 nm, about 90 nm, about 100 nm, about 110 nm, about 120 nm, about 130 nm, about 140 nm, about 150 nm, about 160 nm, or about 170 nm.

It should be noted that the proportion of cationic lipid, cholesterol and modified PEG lipid of the liposome should be formulated within a specific range (for example, including about 40 to 85 mol% of cationic lipid, about 10 to 50 mol% of cholesterol and about 0.001 to 20 mol% of modified PEG lipid) to form the liposome with a nanoparticle structure.

In accordance with some embodiments, the cationic lipid includes 1,2-dioleoyl-3-trimethylammonium propane (DOTAP), 1,2-di-O-octadecenyl-3-trimethylammonium propane (DOTMA), didodecyldimethylammonium bromide (DDAB), 1,2-dioleyloxy-3-dimethylamino propane (DODMA), lipid GL67 (Genzyme Lipid 67), ethyl phosphocholine (ethyl PC), 3β-[N-(N',N'-dimethylaminoethane)-carbamoyl]cholesterol (DC-cholesterol), the derivatives of the foregoing cationic lipid, or a combination of the foregoing cationic lipids and derivatives thereof, but it is not limited thereto.

In accordance with some embodiments, the modified PEG lipid includes DSPE-PEG lipid (1,2-distearoyl-sn-glycero-3-phosphoethanolamine-N-[carboxy(polyethylene glycol)]), DMG-PEG lipid (1,2-dimyristoyl-rac-glycero-3-methoxypolyethylene glycol) or a combination thereof, but it is not limited thereto.

In accordance with some embodiments, the PEG average molecular weight of DSPE-PEG lipid and DMG-PEG lipid is in a range from about 500 to 15000 Da, or from about 800 to 12000 Da, e.g., about 900 Da, about 1000 Da, about 2000 Da, about 3000 Da, about 4000 Da, about 5000 Da, about 6000 Da, about 7000 Da, about 8000 Da, about 9000 Da, about 10000 Da, or about 11000 Da, etc., but the present disclosure is not limited thereto. In accordance with some embodiments, the PEG terminal functional group of DSPE-PEG lipid and DMG-PEG lipid includes amine group ($NH_2$) or maleimide group, but it is not limited thereto.

In accordance with some embodiments, the polymer dispersity index (PDI) of the liposome is less than about 0.4, for example, less than about 0.3, less than about 0.2, or less than about 0.1. PDI can be used to evaluate the breadth of particle size distribution of particles. The larger the PDI, the more particles of various particle sizes exist in the formed liposome, and the worse the uniformity of the liposomes. It should be noted that, in accordance with some embodiments, if the PDI is greater than 0.4, it indicates that the homogeneity of the liposome formulation is poor, and the liposome particles that are formed have poor uniformity.

In accordance with some embodiments, the zeta potential of the liposome is about 0 to 150 mV, or about 5 to 100 mV, but it is not limited thereto. The zeta potential can serve as an indicator of the charged state on the particle surface. Generally, the value of zeta potential between +10mV and -10mV indicates that the particle surface is electrically neutral; if the value of zeta potential is greater than +30mV or less than -30mV, it respectively indicates that the particle surface is positive charged or negatively charged.

In addition, it should be understood that the types of cationic lipids and modified PEG lipids that form the liposome are not limited to those described in the aforementioned embodiments. In accordance with the embodiments of the present disclosure, other suitable types of cationic lipids and modified PEG lipids can be selected, as long as the liposome formed has a suitable nanoparticle structure, e.g., a suitable range of particle size, PDI, etc.

Moreover, in accordance with some embodiments, the immunostimulatory nucleic acid drugs of the immunostimulatory lipoplex includes CpG oligodeoxynucleotide (ODN), small interfering RNA (siRNA), microRNA (miRNA) or a combination thereof.

CpG oligodeoxynucleotides can bind to TLR9 receptor (toll-like receptor 9). CpG oligodeoxynucleotides can strongly activate TLR9, promote the production of interferon, and induce anti-tumor or anti-virus immune response. The CpG oligodeoxynucleotide can be any CpG oligodeoxynucleotide sequence known to be immunostimulatory. In accordance with some embodiments, the sequence length of the CpG oligodeoxynucleotide is from about 15 nucleotides to about 40 nucleotides, but it is not limited thereto. For example, in accordance with some embodiments, the sequence of the CpG oligodeoxynucleotide has at least 85%, for example, 86%, 87%, 88 %, 89%, 90%, 91%, 92%, 93%, 94%, 95%, 96%, 97%, 98%, or 99% similarity to the nucleic acid sequence shown in SEQ ID NO: 1 or 2, but it is not limited thereto.

In accordance with some embodiments, the CpG oligodeoxynucleotide sequence includes one or more unmethylated CpG motifs. In accordance with some embodiments, 85% to 100% of the CpG motifs in the CpG oligodeoxynucleotide sequence are unmethylated. For example, there may be about 88%, 90%, 92%, 95%, or 98% of the CpG motifs are unmethylated, but it is not limited thereto. In accordance with some embodiments, all the CpG motifs in the CpG oligodeoxynucleotide sequence are unmethylated. Moreover, in accordance with some embodiments, the CpG oligodeoxynucleotide sequence includes a modified phosphodiester bond, such as a phosphorothioate bond, thereby reducing the risk of the immunostimulatory nucleic acid drugs being degraded by enzymes in the organism, and improving the stability of the lipoplex. In accordance with some embodiments, 70% to 100%, or about 80% to 100%, for example, 85%, 90% or 95%, of phosphodiester bonds in the CpG oligonucleotide sequence are phosphorothioate bonds, but it is not limited thereto. In accordance with some embodiments, all phosphodiester bonds in the CpG oligonucleotide sequence can be modified to phosphorothioate bonds.

Furthermore, the aforementioned siRNA and miRNA can be any siRNA sequence and miRNA sequence that are known to be immunostimulatory.

In accordance with some embodiments, the particle size of the lipoplex formed by complexation of the liposome and the immunostimulatory nucleic acid drug is in a range from about 50 nm to about 350 nm, or from about 60 nm to about 300 nm, or from about 70 nm to about 250 nm, e.g., about 80 nm, about 90 nm, about 100 nm, about 110 nm, about 120 nm, about 130 nm, about 140 nm, about 150 nm, about 160 nm, about 170 nm, about 180 nm, about 190 nm, about 200 nm, about 210 nm, about 220 nm, about 230 nm, or about 240 nm etc., but the present disclosure is not limited thereto.

In accordance with some embodiments, the polymer dispersity index (PDI) of the lipoplex formed by complexation of the liposome and the immunostimulatory nucleic acid drug is less than about 0.4, for example, less than about 0.3, less than about 0.2, or less than about 0.1. It should be noted that, in accordance with some embodiments, if the PDI is greater than 0.4, it means that the homogeneity of the lipoplex formulation is poor, and the lipoplex particles that are formed have poor uniformity.

In accordance with some embodiments, the zeta potential of the lipoplex is about -70 mV to about 150 mV, or about -60 mV to about 100 mV, but it is not limited thereto.

It should be noted that, in accordance with the embodiments of the present disclosure, the immunostimulatory lipoplex with specific composition can improve the problems of poor stability and insufficient tissue exposure of immunostimulatory nucleic acid drugs, and increase the specific immune activation of the drugs

Furthermore, in accordance with some embodiments, the aforementioned immunostimulatory lipoplexes can be used for the preparation of medicines for the treatment of cancer. In accordance with some embodiments, the immunostimulatory lipoplex further includes a pharmaceutically acceptable carrier. For example, in accordance with some embodiments, an effective amount of the aforementioned immunostimulatory lipoplex can be administered to an individual in need thereof. In accordance with some embodiments, the individual may include a mammal, e.g., mouse, rat, guinea pig, rabbit, dog, cat, monkey, orangutan or human, but it is not limited thereto. In accordance with some embodiments, the individual is a human. In accordance with some embodiments, the means of administering the medicine including the immunostimulatory lipoplex to the individual may include intravenous injection, subcutaneous injection, intramuscular injection, or inhalation, but it is not limited thereto.

In accordance with some embodiments, the aforementioned cancers may include colon cancer, breast cancer, lung cancer, pancreatic cancer, liver cancer, stomach cancer, esophageal cancer, head and neck squamous cell carcinoma, prostate cancer, bladder cancer, lymphoma, gallbladder cancer, kidney cancer, blood cancer, colorectal cancer, multiple myeloma, ovarian cancer, cervical cancer or glioma, but it is not limited thereto.

It should be noted that, in accordance with some embodiments of the present disclosure, the immunostimulatory lipoplex can be administered in a systemic manner, which can improve the problem of drug delivery limitations of general intratumoral injection, for example, improve the problem of poor efficacy in deep tumors.

In addition, in accordance with some embodiments, the immunostimulatory lipoplex can be used in combination with an immune checkpoint inhibitor, and the immune checkpoint inhibitor includes anti-PD-1/PD-L1 antibody, anti-CTLA-4 antibody, or a combination thereof, but it is not limited thereto. The combined use of the immunostimulatory lipoplex and the current immune checkpoint inhibitor can exert a synergistic effect to further improve the efficacy of cancer immunotherapy.

In accordance with some embodiments, a pharmaceutical composition is provided, the pharmaceutical composition includes the aforementioned immunostimulatory lipoplex, and the pharmaceutical composition can be used for the treatment of cancer. The pharmaceutical composition may further include a pharmaceutically acceptable carrier. In accordance with some embodiments, the pharmaceutical composition may further include an immune checkpoint inhibitor, which is used in combination with the immunostimulatory lipoplex. The immune checkpoint inhibitor may include anti-PD-1/PD-L1 antibody, anti-CTLA-4 antibody or a combination thereof, but it is not limited thereto. In accordance with some embodiments, the manner in which the pharmaceutical composition is administered to the individual may include intravenous injection, subcutaneous injection, intramuscular injection, or inhalation, but it is not limited thereto.

In accordance with some embodiments, the pharmaceutical composition may present in a form of solution or suspension. Alternatively, the pharmaceutical composition may be a dehydrated solid (e.g., a lyophilized or spray-dried solid). In accordance with some embodiments, the pharmaceutical composition is sterile and non-toxic to the individual. Moreover, in accordance with some embodiments, the aforementioned pharmaceutically acceptable carrier includes excipients, solubilizers, buffers, stabilizers or preservatives, but it is not limited thereto.

For example, the excipient may include a solvent. In accordance with some embodiments, the pharmaceutical composition includes an aqueous vehicle as a solvent. The aqueous vehicle may include, for example, sterile water, saline solution, phosphate buffered saline, or Ringer's solution, but it is not limited thereto. In accordance with some embodiments, the solubilizer is a protective agent that helps stabilize the immunostimulatory lipoplex and prevent its degradation during lyophilization or spray-drying and/or during storage. The solubilizer may include, for example, sugars (monosaccharides, disaccharides, and polysaccharides), such as sucrose, lactose, trehalose, mannitol, sorbitol, or glucose, but it is not limited thereto. Furthermore, in accordance with some embodiments, the buffer can control the pH value to prevent degradation of the immunostimulatory lipoplex during processing, storage, and the like. For example, the buffer may include salts such as acetate, citrate, phosphate, or sulfate, but it is not limited thereto. For example, the buffer may also include amino acids, such as arginine, glycine, histidine or lysine, but it is not limited thereto. In accordance with some embodiments, the stabilizer may include, for example, dextrose, glycerol, sodium chloride, glycerol, or mannitol, but it is not limited thereto. In accordance with some embodiments, the preservative may include, for example, an antioxidant or an antimicrobial agent, but it is not limited thereto.

In addition, in accordance with some embodiments of the present disclosure, a kit is provided, and the kit includes the aforementioned pharmaceutical composition and an instruction describing the method of use. The kit including the pharmaceutical composition is appropriately packaged. In accordance with some embodiments, the kit may further include a device for administering the pharmaceutical composition (e.g., syringe and needle, nebulizer, or dry powder inhalation device, etc.).

In order to make the above-mentioned and other purposes, features and advantages of the present disclosure more thorough and easy to understand, a number of preparation examples, examples and comparative examples are given below, and are described in detail as follows, but they are not intended to limit the scope of the present disclosure.

**Preparative Example 1: preparation of liposome**

According to formula Nos. F1 to F25 and N1 to N11 shown in following Table 1, cationic lipid, cholesterol and modified PEG lipid were weighed and added into a round-bottom flask, and the lipids were dissolved with methanol (purchased from Merck). The cationic lipid DOTAP used was purchased from Avanti (CAS No. 132172-61-3), the cholesterol was purchased from Nippon Fine Chemical (CAS No. 57-88-5), and the modified PEG lipid DSPE-PEG2000 (DSPE-PEG-2K) was purchased from Nippon Fine Chemical (CAS No. 247925-28-6). DSPE-PEG-1K, 5K and 10K were purchased from Nanocs; DSPE-PEG-2K-NH$_2$ was purchased from Avanti (CAS No. 474922-26-4); and DSPE-PEG-2K-Maleimide was purchased from Avanti (CAS No. 474922-22-0).

After confirming that the lipids were all dissolved, the round-bottom flask was connected to a buffer bottle and a vacuum concentrator and placed in a 60°C water bath, and the temperature was equilibrated with a rotating speed of 150 rpm. Next, the vacuum degree was set to 150 mPa, the vacuum pump was turned on and operated for about 5 minutes. Subsequently, the vacuum degree was set to 20 mPa, the solvent was completely drained, and the formulated mixture formed a thin film. Next, 10 mM Tris buffer (pH 7.5, purchased from VWR Life Science) was added to the round-bottom flask for hydration, and the thin film was completely dissolved by low-power ultrasonic vibration. Afterwards, the hydrated solution was subjected to particle-size classification (sizing) by high-power ultrasonic vibration (Pulse sonicator) or high-pressure homogenizer (microfluidizer). If the sample was transparent and clear, it was then filtered with a 0.22 μm filter and divided into fractions; if the sample was precipitated, then the sample was directly transferred to a centrifuge tube and stored at 4°C.

Then, 20 μL of the sample was taken and added to 980 μL of PBS. After the mixture was shaken evenly, the particle diameter of the liposomes formed by each formula was measured with Zetasizer Nano ZS (Malvern Panalytical). In addition, 20 μL of the sample was added to 980 μL of 10 mM NaCl solution, shaken evenly, and the zeta potential of the liposomes formed by each formula was measured with Zetasizer Nano ZS (Malvern Panalytical).

Table 1

| Formula No. | DOTAP mol% | Cholesterol mol% | PEG lipid mol% | Average particle diameter (nm) | PDI | Zeta potential (mV) | PEG lipid type |
|---|---|---|---|---|---|---|---|
| F1 | 67.24 | 22.09 | 10.67 | 89.2 | 0.176 | 11.8 | DSPE-PEG-2K |
| F2 | 60.35 | 39.65 | 0 | 164.7 | 0.276 | 98.7 | DSPE-PEG-2K |
| F3 | 58.47 | 38.59 | 2.66 | 97.1 | 0.209 | 28.3 | DSPE-PEG-2K |
| F4 | 57.22 | 37.59 | 5.19 | 124.1 | 0.242 | 21.0 | DSPE-PEG-2K |
| F5 | 55.77 | 36.64 | 7.59 | 160.8 | 0.35 | 17.7 | DSPE-PEG-2K |
| F6 | 50.37 | 49.63 | 0 | 164.8 | 0.279 | 93.4 | DSPE-PEG-2K |
| F7 | 49.24 | 48.52 | 2.23 | 101.7 | 0.24 | 37.8 | DSPE-PEG-2K |
| F8 | 48.17 | 47.46 | 4.37 | 100.2 | 0.227 | 24.7 | DSPE-PEG-2K |
| F9 | 47.14 | 46.45 | 6.41 | 136.3 | 0.323 | 20.6 | DSPE-PEG-2K |
| F10 | 75.27 | 24.73 | 0 | 131.9 | 0.265 | 98.1 | DSPE-PEG-2K |
| F11 | 74.51 | 24.48 | 1.01 | 84.32 | 0.213 | 36.2 | DSPE-PEG-2K |

(continued)

| Formula No. | DOTAP | Cholesterol | PEG lipid | Average particle diameter (nm) | PDI | Zeta potential (mV) | PEG lipid type |
|---|---|---|---|---|---|---|---|
| | mol% | mol% | mol% | | | | |
| F12 | 72.79 | 23.91 | 3.3 | 89.99 | 0.199 | 25.3 | DSPE-PEG-2K |
| F13 | 70.46 | 23.15 | 6.39 | 91.39 | 0.207 | 17.1 | DSPE-PEG-2K |
| F14 | 68.28 | 22.43 | 9.29 | 89.51 | 0.168 | 12.2 | DSPE-PEG-2K |
| F15 | 81.48 | 17.32 | 1.2 | 81.67 | 0.242 | NA | DSPE-PEG-2K |
| F16 | 75.22 | 19.41 | 5.36 | 78.96 | 0.257 | NA | DSPE-PEG-2K |
| F17 | 56.71 | 25.61 | 17.68 | 103.8 | 0.245 | NA | DSPE-PEG-2K |
| F18* | 67.24 | 22.09 | 10.67 | 73.16 | 0.164 | 10.4 | DSPE-PEG-2K |
| Formula No. | DOTAP | Cholesterol | PEG lipid | Average particle diameter (nm) | PDI | Zeta potential (mV) | PEG lipid type |
| | mol% | mol% | mol% | | | | |
| F19 | 67.21 | 22.08 | 10.71 | 87.20 | 0.167 | 32.5 | DSPE-PEG-2K-NH$_2$ |
| F20 | 67.59 | 22.20 | 10.21 | 94.09 | 0.154 | 8.52 | DSPE-PEG-2K-Mal |
| F21 | 74.09 | 24.34 | 1.57 | 77.63 | 0.226 | 53.1 | DSPE-PEG-1K |
| F22 | 74.90 | 24.60 | 0.50 | 65.06 | 0.196 | 49.4 | DSPE-PEG-5K |
| F23 | 75.07 | 24.66 | 0.27 | 64.75 | 0.220 | 25.4 | DSPE-PEG-10K |
| F24 | 74.42 | 24.45 | 1.13 | 82.21 | 0.256 | 38.0 | DMG-PEG-2K |
| F25 | 67.24 | 22.09 | 10.67 | 59.61 | 0.171 | 12.3 | DSPE-PEG-2K |
| N1 | 28.13 | 50.82 | 21.05 | precipitated | | | DSPE-PEG-2K |
| N2 | 38.54 | 9.95 | 51.51 | 25.26 | 0.298 | -6.82 | DSPE-PEG-2K |
| N3 | 35.63 | 64.37 | 0.00 | precipitated | | | DSPE-PEG-2K |
| N4 | 41 | 59 | 0 | precipitated | | | DSPE-PEG-2K |
| N5 | 10 | 40 | 50 | precipitated | | | DSPE-PEG-2K |

(continued)

| Formula No. | DOTAP | Cholesterol | PEG lipid | Average particle diameter (nm) | PDI | Zeta potential (mV) | PEG lipid type |
|---|---|---|---|---|---|---|---|
| | mol% | mol% | mol% | | | | |
| N6 | 15 | 70 | 15 | precipitated | | | DSPE-PEG-2K |
| N7 | 34 | 65 | 1 | precipitated | | | DSPE-PEG-2K |
| N8 | 12 | 80 | 8 | precipitated | | | DSPE-PEG-2K |
| N9 | 33 | 48 | 19 | precipitated | | | DSPE-PEG-2K |
| N10 | 41 | 58 | 1 | precipitated | | | DSPE-PEG-2K |
| N11 | 41 | 15 | 44 | 36.23 | 0.239 | -4.1 | DSPE-PEG-2K |
| *An additional excipient, 2-Hydroxypropyl-beta-cyclodextrin (HP-beta-CD), was added to the formula with an amount of 0.82 mg/mL. | | | | | | | |

As shown in Table 1, the range of particle diameters of the liposomes formed by formula Nos. F1 to F25 was from 50 nm to 200 nm, and the PDI values of the liposomes were all less than 0.4, and most of them were less than 0.3, which represents that the liposome particles have good homogeneity and uniformity. Furthermore, the particle surfaces of the liposomes formed by formula Nos. F1 to F25 were mostly positively charged. In contrast, formula Nos. N1 and N3 to N10 caused precipitation and could not form liposomes with a particle structure, and the liposomes formed by formulas N2 and N11 had a smaller particle size range (less than 50 nm), and particle surfaces of the liposomes were negatively charged.

**Preparative Example 2: preparation of lipoplex**

First, the frozen crystal powders of CpG oligodeoxynucleotides (CpG-ODN), CpG-7909 (the nucleic acid sequence shown in SEQ ID NO: 1, entrusted to Integrated DNA Technologies for custom synthesis) or CpG-30-PS (the nucleic acid sequence shown in SEQ ID NO: 2, entrusted to Integrated DNA Technologies for custom synthesis), were dissolved with 10 mM Tris buffer solution (pH 7.5, purchased from VWR Life Science) and slightly shaken to confirm that the frozen crystal powders of CpG-ODN were completely dissolved. In addition, the solution of CpG-ODN was then filtered using a 0.22 μm filter.

Thereafter, 10 mM Tris buffer solution (pH 7.5) was added into a sample bottle or centrifuge tube, and according to formula Nos. C1 to C20 and E1 to E2 shown in following Table 2, the liposome (liposome was prepared as described in Preparative Example 1) was added first, and then the CpG-ODN solution was added. After mixing the samples, stirring was continued for 30 to 40 minutes to complete the complexation of the liposome with the oligodeoxynucleotide to form a lipoplex.

Then, 20 μL of the sample was taken and added to 980 μL of PBS. After the mixture was shaken evenly, the particle diameter of lipoplexes formed by each formula was measured with Zetasizer Nano ZS (Malvern Panalytical). In addition, 20 μL of the sample was added to 980 μL of 10 mM NaCl solution, shaken evenly, and the zeta potential of lipoplexes formed by each formula was measured with Zetasizer Nano ZS (Malvern Panalytical).

Table 2

| Formula No. | DOTAP mol% | Cholesterol mol% | PEG lipid mol% | CpG-7909 N/P ratio | CpG-7909 content (μM) | Average particle diameter (nm) | PDI | Zeta potential (mV) | PEG lipid type |
|---|---|---|---|---|---|---|---|---|---|
| C1 | 67.24 | 22.09 | 10.67 | 4.8 | 64.95 | 83.37 | 0.169 | 6.90 | DSPE-PEG-2K |
| C2 | 72.79 | 23.91 | 3.30 | 4.8 | 64.95 | 121.90 | 0.163 | 14.90 | DSPE-PEG-2K |
| C3 | 58.47 | 38.59 | 2.66 | 4.8 | 64.95 | 147.20 | 0.197 | 20.00 | DSPE-PEG-2K |
| C4 | 67.24 | 22.09 | 10.67 | 4.8 | 64.95 | 88.00 | 0.148 | 31.40 | DSPE-PEG2000-NH₂ |
| C5 | 67.24 | 22.09 | 10.67 | 4.8 | 64.95 | 103.90 | 0.147 | 1.45 | DSPE-PEG2000-Mal |
| C6* | 67.24 | 22.09 | 10.67 | 4.8 | 64.95 | 71.71 | 0.150 | 9.14 | DSPE-PEG-2K |
| C7 | 66.87 | 21.96 | 10.62 | 4.8 | 64.95 | 60.10 | 0.152 | 4.24 | DSPE-PEG-2K |
| C8 | 74.51 | 24.48 | 1.01 | 4.8 | 19.48 | 148.5 | 0.168 | 19.5 | DSPE-PEG-2K |
| C9 | 72.79 | 23.91 | 3.30 | 4.8 | 19.48 | 111.2 | 0.170 | 14.5 | DSPE-PEG-2K |
| C10 | 67.24 | 22.09 | 10.67 | 4.8 | 19.48 | 85.81 | 0.173 | 8.09 | DSPE-PEG-2K |
| C11 | 74.09 | 24.34 | 1.57 | 4.8 | 19.48 | 206.5 | 0.140 | 34.7 | DSPE-PEG-1K |
| C12 | 74.90 | 24.60 | 0.50 | 4.8 | 19.48 | 248.9 | 0.167 | 44.4 | DSPE-PEG-5K |
| C13 | 75.07 | 24.66 | 0.27 | 4.8 | 19.48 | 174.0 | 0.099 | 14.5 | DSPE-PEG-10K |
| C14 | 74.42 | 24.45 | 1.13 | 4.8 | 19.48 | 148.6 | 0.155 | 30.4 | DMG-PEG-2K |
| C15 | 75.27 | 24.73 | 0 | 4.8 | 64.95 | 302.10 | 0.305 | 70.50 | DSPE-PEG-2K |
| C16 | 74.51 | 24.48 | 1.01 | 1.2 | 19.48 | 186.70 | 0.099 | -6.47 | DSPE-PEG-2K |
| C17 | 72.79 | 23.91 | 3.30 | 1.2 | 19.48 | 121.90 | 0.162 | -10.10 | DSPE-PEG-2K |
| C18 | 67.24 | 22.09 | 10.67 | 1.2 | 19.48 | 96.91 | 0.197 | -12.00 | DSPE-PEG-2K |
| C19 | 72.79 | 23.91 | 3.30 | 4.8 | 6.49 | 118.50 | 0.148 | 14.70 | DSPE-PEG-2K |
| E1 | 41 | 15 | 44 | 4.8 | 19.48 | 36.81 | 0.308 | -7.62 | DSPE-PEG-2K |

(continued)

| Formula No. | DOTAP | Cholesterol | PEG lipid | CpG-7909 | CpG-7909 content ($\mu$M) | Average particle diameter (nm) | PDI | Zeta potential (mV) | PEG lipid type |
|---|---|---|---|---|---|---|---|---|---|
| | mol% | mol% | mol% | N/P ratio | | | | | |
| E2 | 38.54 | 9.95 | 51.51 | 4.0 | 19.48 | 77.98 | 0.369 | -6.43 | DSPE-PEG-2K |

| Formula No. | DOTAP | Cholesterol | PEG lipid | CpG-30-PS | CpG-30-PS content ($\mu$M) | Average particle diameter (nm) | PDI | Zeta potential (mV) | PEG lipid type |
|---|---|---|---|---|---|---|---|---|---|
| | mol% | mol% | mol% | N/P ratio | | | | | |
| C20 | 67.24 | 22.09 | 10.67 | 4.8 | 10.34 | 122.2 | 0.209 | 7.58 | DSPE-PEG-2K |

*An additional excipient, 2-Hydroxypropyl-beta-cyclodextrin (HP-beta-CD), was added to the formula with an amount of 0.82 mg/mL.

As shown in Table 2, the range of particle diameters of the lipoplexes formed by formula Nos. C1 to C20 was from 50 nm to 300 nm, and the PDI values of the lipoplexes were all less than 0.35, and most of them were less than 0.2, which represents that the lipoplex particles have good homogeneity and uniformity. Furthermore, the particle surfaces of the lipoplexes can be positively or negatively charged.

**Example 1: pharmacokinetic evaluation of lipoplex**

Analysis of the drug exposure of lipoplex (liposome complexed with CpG oligodeoxynucleotide) and CpG oligodeoxynucleotide alone (CpG-ODN) in animals by pharmacokinetic assessment in mice was performed to confirm that the lack of drug exposure to the target tissue (such as tumor) was the main reason for affecting the efficacy of the drug.
First, the liposome of formula F12 was added into a sample bottle or centrifuge tube, then $^{125}$I (N-Succinimidyl 4-methyl-3-trimethylstannyl benzoate, CENTRIPure MINI Spin Columns) labeled CpG-7909 (5 mg/mL in ddH2O, pH 7.5) was added for complex reaction. The complex reaction was carried out in two steps, and the solutions of each step were mixed and continuously stirred for 15 to 20 minutes. The complex volume in the two steps are shown in Table 3 below, which was prepared at a drug concentration of 0.5 mg/mL required for *in vivo* pharmacokinetic testing (dose was 50 $\mu$g/mouse, and dosing volume was 100 $\mu$L/mouse). After the complex reaction was completed, the particle size, PDI and zeta potential of the formed lipoplex were measured. The particle size was 89.81 nm, the PDI was 0.205, and the zeta potential was 24.3 mV.

Table 3

| Total volume 1.2 mL | $^{125}$I labelled CpG-7909 (5 mg/mL) | Lipoplex of formula 12 |
|---|---|---|
| First step | 40 $\mu$L | 720 $\mu$L |
| Second step | 80 $\mu$L | 360 $\mu$L |

Then, the lipoplex of formula F12 or $^{125}$I labeled CpG-7909 (CpG-ODN) was intravenously injected into mice (female Balb/c mice, weighing approximately 25 g, were purchased from Lesco Biotechnology) at a single dose, with the dose of 50 $\mu$g/mouse and the injection volume of 100 $\mu$L/mouse. The drug groups were divided into Lipoplex group and CpG-ODN group, and each drug group was divided into blood collection (PK) group and bio-distribution group. In the blood collection group (3 mice), blood was collected at 10 minutes, 30 minutes, 1 hour, 2 hours, 4 hours, 8 hours, 24 hours, 3 days and 7 days after administration. In the bio-distribution group (3 mice), liver, kidney, spleen, and lymph node were collected at 4 hours, 24 hours, 3 days, and 7 days after administration. In addition, the gamma counter was used to analyze the activity of the target substance. The experimental results of the blood collection group are shown in Table 4, and the experimental results of the bio-distribution group are shown in FIG. 1 and Table 5 (quantification results).

Table 4

|  | CpG-ODN group | Lipoplex group |
|---|---|---|
| Drug exposure (hr*ng/mL) | 9183.9±585.8 | 16093.1±2398.9 |

Table 5

|  | Fold comparison of CpG-ODN concentration in tissues between Lipoplex group and CpG-ODN group |
|---|---|
| Liver | 1.7 |
| Spleen | 166.4 |
| Kidney | 1.8 |
| Lymph node | 1.7 |

As shown in Table 4, the Lipoplex group can increase the CpG-ODN exposure in the blood by about 2 times. Furthermore, as shown in the results of FIG. 1 and Table 5, the Lipoplex group increased the CpG-ODN exposure in tissues by about 1.7 to 166 times. It can be seen that, compared with the CpG oligodeoxynucleotide used alone, the lipoplex can improve the pharmacokinetic properties of CpG oligodeoxynucleotides *in vivo.*

**Example 2: evaluation of TLR9 activation ability of lipoplex**

The HEK-Blue™ hTLR9 cell line (purchased from InvivoGen, product number hkb-htlr9) expressing human TLR9 was used to evaluate TLR9 activation ability of the lipoplex of formula C1 (Lipoplex group) and CpG-7909 alone (CpG-ODN group). Specifically, the HEK-Blue™ hTLR9 cell line can produce secreted embryonic alkaline phosphatase (SEAP) after induction and activation, which can be used to evaluate TLR9 activity induced by Lipoplex group and CpG-ODN group. The Phospha-Light™ SEAP Reporter Gene Assay System (purchased from Thermo Fisher Scientific, product number T1017) was used to detect SEAP in the cell culture supernatants treated with Lipoplex group and CpG-ODN group. Two days before induction, the cell culture supernatants of the HEK-Blue™ hTLR9 cells seeded in the 96-well plate were removed, and complete cell culture medium containing different inducers was added. After culturing in a cell incubator for another 7 hours, the samples of cell culture supernatants were collected and stored at - 80°C. After thawing the samples stored at -80°C, the SEAP reporter gene activity in the cell culture supernatant was analyzed by using the above SEAP Reporter Gene Assay System, and then the relevant analysis data was processed with the GraphPad Prism biostatistics software. The results are shown in FIG. 2.
As shown in FIG. 2, the experimental results show that the $EC_{50}$ value of CpG-ODN group was about 485 nM, and the $EC_{50}$ value of Lipoplex group was about 46 nM. Compared with the CpG oligodeoxynucleotide used alone, the lipoplex formulation can greatly enhance the activation ability of TLR9 ($EC_{50}$ value increased by about 10 times).

**Example 3: evaluation of TLR9 activation ability of lipoplex**

The HEK293-hTLR9/NF-κB-luc cell line (purchased from BPS Bioscience, product number 60685) expressing human TLR9 was used to evaluate TLR9 activation ability of the lipoplexes of formula C1, C3, C2, C6 (respectively corresponding Lipoplex group-1. Lipoplex group-2, Lipoplex group-3, Lipoplex group-4) and CpG-7909 alone (CpG-ODN group). Specifically, the HEK293-hTLR9/NF-κB-luc cell line can produce firefly luciferase (FLuc) after induction and activation, which can be used to evaluate TLR9 activity induced by Lipoplex groups-1 to 4 and CpG-ODN group. Rapid Detection of Firefly Luciferase Activity (purchased from Promega, product number E4550) combined with CelLytic M Cell Lysis Reagent (purchased from Merck KGaA, product number C2978) were used to detect the reporter gene activity in the cells treated with Lipoplex groups-1 to 4 and CpG-ODN group. Two days before induction, the cell culture supernatants of the HEK293-hTLR9/NF-κB-luc cells seeded in the 96-well plate were removed, and complete cell culture medium containing different inducers was added. After culturing in a cell incubator for another 6 hours, the lysis buffer of CelLytic M Cell Lysis Reagent was added, and the above Rapid Detection of Firefly Luciferase Activity luminescent reporter gene kit was used to analyze the reporter gene activity in the cell lysate. Afterwards, relevant data processing and analysis were performed with GraphPad Prism biostatistics software, and the results are shown in FIG. 3.
As shown in FIG. 3, the experimental results show that the $EC_{50}$ value of CpG-ODN group was about 242 nM, the $EC_{50}$ value of Lipoplex group-1 was about 18 nM, and the $EC_{50}$ value of Lipoplex group-2 was about 20 nM, the $EC_{50}$ value of Lipoplex group-3 was about 9 nM, and the $EC_{50}$ value of Lipoplex group-4 was about 12 nM. Compared with CpG oligodeoxynucleotide used alone, Lipoplex group-1 to 4 with different formulations can greatly enhance the activation ability of TLR9 ($EC_{50}$ value increased by about 10 times).

**Example 4: evaluation of cytokine activation ability of lipoplex**

First, Ficoll-Paque PREMIUM 1.077 (purchased from GE Healthcare) was used to isolate human blood. The peripheral blood mononuclear cells (PBMC) were isolated after centrifugation at 400 ×g for 40 minutes, and the PBMC cells were collected and then washed and centrifuged with DPBS buffer. The platelets were isolated after centrifugation at 100 ×g for 10 minutes, and then seeded in a 48-well plate at $1.5 \times 10^6$ of cells. Next, the serially diluted different concentrations of CpG-7909 (CpG-ODN group) or lipoplex of formula C1 (Lipoplex group) were added to the culture plate and cultured for 24 hours. Then, the supernatants were collected, and Bio-Plex Pro™ Human Cytokine 27-plex Assay (purchased from Bio-rad) was used to determine the concentration changes of IFN-γ and IL-6. In addition, IFN-α concentration was determined using PBL VeriKine-HS Mouse IFN-α All Subtype ELISA Kit Product (purchased from PBL assay science, #42115).

As shown in FIGs. 4A to 4C, the experimental results show that Lipoplex group can promote human PBMC cells to produce IL-6 and IFN-γ at low concentrations (for example, about 10 nM), and the activation ability is higher than that of CpG-ODN group. In addition, IFN-α, which was hardly activated in CpG-ODN group, also had promoted secretion in Lipoplex group. Thus, it can be seen that Lipoplex group can effectively promote the secretion of cytokines related to immune and anti-cancer.

**Example 5: evaluation of immune anticancer effect of lipoplex *in vivo***

The mouse colorectal cancer cell line CT26 (purchased from ATCC) was cultured in RPMI 1640 medium (purchased from Thermo Fisher Scientific) supplemented with 10% fetal bovine serum (FBS), and placed in the incubator at 37°C, 5% $CO_2$. The CT26 cell line was inoculated to the right back subcutaneous of 5 to 8-week-old mice (female BALB/c mice, purchased from Lesco Biotechnology Co., Ltd.) with $2 \times 10^5$ cells. When the average volume of tumors reached 100 to 200 $mm^3$, the administration was carried out according to the following groups: Control group, Anti-PD-1 antibody group, CpG-ODN group, Lipoplex group (BIW), Anti-PD-1 antibody + CpG-ODN group, Anti-PD-1 antibody + Lipoplex group (QW), Anti-PD-1 antibody + Lipoplex group (BIW).

Control group was intravenously injected with normal saline twice a week, and the injection volume was 100 μL. Anti-PD-1 antibody group was intraperitoneally injected with anti-mouse PD-1 antibody (purchased from Bio X Cell, product number BE0146) twice a week , the injection dose was 250 μg, and the injection volume was 100 μL. CpG-ODN group was intravenously injected with CpG-7909 twice a week, the injection dose was 50 μg, and the injection volume was 100 μL. Lipoplex group (BIW) was intravenously injected with the of formula C1 twice a week, the injection dose was 50 μg, and the injection volume was 100 μL. The anti-mouse PD-1 antibody and CpG-7909 were used in combination in the administration of Anti-PD-1 antibody + CpG-ODN group, among which the anti-PD-1 antibody was intraperitoneally injected twice a week with the dose of 250 μg and the volume of 100 μL, and CpG-7909 was intravenously injected twice a week with the dose of 50 μg and the volume of 100 μL. The anti-mouse PD-1 antibody and the lipoplex of formula C1 were used in combination in the administration of Anti-PD-1 antibody + Lipoplex group (QW), among which the anti-PD-1 antibody was intraperitoneally injected twice a week with the dose of 250 μg and the volume of 100 μL, and the lipoplex was intravenously injected once a week with the dose of 50 μg and the volume of 100 μL. The anti-mouse PD-1 antibody and the lipoplex of formula C1 were used in combination in the administration of Anti-PD-1 antibody + Lipoplex group (BIW), among which the anti-PD-1 antibody was intraperitoneally injected twice a week with the dose of 250 μg and the volume of 100 μL, and the lipoplex was intravenously injected twice a week with the dose of 50 μg and the volume of 100 μL. The period of administration was 14 days. 6 mice in each group were used for the experiment (n=6), and the tumor volume was measured twice a week. The results are shown in FIG. 5. In addition, the tumor growth inhibition value (TGI) was calculated using the following formula (A)

$$(1 - [(Tn)/(Cn)]) \times 100\%\dots\text{Formula (A)}$$

, wherein Tn is the tumor volume of the treatment group on day n, and Cn is the tumor volume of the control group on day n.

Table 6

| Tumor growth inhibition value (TGI) (day 11 to day 25) (Mean ± SEM) (n=6) | |
| --- | --- |
| Control group | -- |
| Anti-PD-1 antibody group | 30 ± 11% |

(continued)

| Tumor growth inhibition value (TGI) (day 11 to day 25) (Mean $\pm$ SEM) (n=6) | |
| --- | --- |
| CpG-ODN group | 40 $\pm$ 12% |
| Lipoplex group (BIW) | 32 $\pm$ 22% |
| Anti-PD-1 antibody + CpG-ODN group | 38 $\pm$ 15% |
| Anti-PD-1 antibody + Lipoplex group (QW) | 78 $\pm$ 8% |
| Anti-PD-1 antibody + Lipoplex group (BIW) | 76 $\pm$ 18% |

As shown in FIG. 5 and Table 6, the experimental results show that Anti-PD-1 antibody + Lipoplex group (QW) and Anti-PD-1 antibody + Lipoplex group (BIW) that used anti-PD-1 antibody and lipoplex in combination could exert a synergistic effect with the same dose, and had a better tumor inhibitory effect (TGI). In addition, the administration frequency of once a week (QW) and twice a week (BIW) had no significant difference in efficacy.

**Example 6: evaluation of immune anticancer effect of lipoplex *in vivo***

Example 6 evaluates the influence of different formulations of lipoplexes (different composition ratios) and lowering the administration dose on immune anticancer effect. The same method was used as that in Example 5, $2 \times 10^5$ cells of CT26 cell line were inoculated to the right back subcutaneous of 5 to 8-week-old mice (female BALB/c mice, purchased from Lesco Biotechnology Co., Ltd.). When the average volume of tumors reached 100 to 200 mm$^3$, the administration was carried out according to the following groups: Control group, Anti-PD-1 antibody + Lipoplex group-1, Anti-PD-1 antibody + Lipoplex group-2, and Anti-PD-1 antibody + Lipoplex group-3.

Control group was intravenously injected with normal saline twice a week, and the injection volume was 100 $\mu$L. The anti-mouse PD-1 antibody and the lipoplex of formula C10 were used in combination in the administration of Anti-PD-1 antibody + Lipoplex group-1, among which the anti-PD-1 antibody was intraperitoneally injected twice a week with the dose of 250 $\mu$g and the volume of 100 $\mu$L, and the lipoplex was intravenously injected once a week with the dose of 15 $\mu$g and the volume of 100 $\mu$L. The anti-mouse PD-1 antibody and the lipoplex of formula C9 were used in combination in the administration of Anti-PD-1 antibody + Lipoplex group-2, among which the anti-PD-1 antibody was intraperitoneally injected twice a week with the dose of 250 $\mu$g and the volume of 100 $\mu$L, and the lipoplex was intravenously injected once a week with the dose of 15 $\mu$g and the volume of 100 $\mu$L. The anti-mouse PD-1 antibody and the lipoplex of formula C8 were used in combination in the administration of Anti-PD-1 antibody + Lipoplex group-3, among which the anti-PD-1 antibody was intraperitoneally injected twice a week with the dose of 250 $\mu$g and the volume of 100 $\mu$L, and the lipoplex was intravenously injected once a week with the dose of 15 $\mu$g and the volume of 100 $\mu$L. The period of drug administration was 14 days. 8 mice in each group were used for the experiment (n=8), and the tumor volume was measured twice a week. The results are shown in FIG. 6. In addition, the tumor growth inhibition value of each group was calculated, and the results are shown in Table 7.

Table 7

| Tumor growth inhibition value (TGI) (day 11 to day 25) (Mean $\pm$ SEM) (n=8) | |
| --- | --- |
| Control group | -- |
| Anti-PD-1 antibody + Lipoplex group-1 | 73 $\pm$ 12% |
| Anti-PD-1 antibody + Lipoplex group-2 | 85 $\pm$ 14% |
| Anti-PD-1 antibody + Lipoplex group-3 | 96 $\pm$ 9% |

As shown in FIG. 6 and Table 7, the experimental results show that Anti-PD-1 antibody + Lipoplex groups-1 to 3 that used anti-PD-1 antibody and lipoplexes of different formulations in combination all have good tumor inhibitory effect (TGI). There was no significant difference in the efficacy in the three groups of different lipoplex formulations. Furthermore, when the lipoplexes were used in combination with anti-PD-1 antibodies, the tumor inhibitory effect would not be affected even if the total dose was reduced. However, it is worth noting that since the dose is reduced, the concern of side effects caused by the original active ingredient can be reduced.

**Comparative Example 1: evaluation of immune anticancer effect of lipoplex *in vivo***

Comparative Example 1 evaluates the influence of different formulations of lipoplexes (different composition ratios) and lowering the administration dose on immune anticancer effect. The same method was used as that in Example 5, $2\times10^5$ cells of CT26 cell line were inoculated to the right back subcutaneous of 5 to 8-week-old mice (female BALB/c mice, purchased from Lesco Biotechnology Co., Ltd.). When the average volume of tumors reached 100 to 200 mm$^3$, the administration was carried out according to the following groups: Control group, Anti-PD-1 antibody group, Anti-PD-1 antibody + Lipoplex group.

Control group was intravenously injected with normal saline twice a week, and the injection volume was 100 $\mu$L. Anti-PD-1 antibody group was intraperitoneally injected with anti-mouse PD-1 antibody (purchased from Bio X Cell, product number BE0146) twice a week , the injection dose was 250 $\mu$g, and the injection volume was 100 $\mu$L. The anti-mouse PD-1 antibody and the lipoplex of formula E2 were used in combination in the administration of Anti-PD-1 antibody + Lipoplex group, among which the anti-PD-1 antibody was intraperitoneally injected twice a week with the dose of 250 $\mu$g and the volume of 100 $\mu$L, and the lipoplex was intravenously injected once a week with the dose of 15 $\mu$g. The period of drug administration was 24 days. 8 mice in each group were used for the experiment (n=8), and the tumor volume was measured twice a week. The results are shown in FIG. 7. In addition, the tumor growth inhibition value of each group was calculated, and the results are shown in Table 8.

Table 8

| Tumor growth inhibition value (TGI) (day 10 to day 34) (Mean $\pm$ SEM) (n=8) | |
| --- | --- |
| Control group | -- |
| Anti-PD-1 antibody group | 41 $\pm$ 17% |
| Anti-PD-1 antibody + Lipoplex group | 54 $\pm$ 11% |

As shown in FIG. 7 and Table 8, the experimental results show that compared with Anti-PD-1 antibody group that used the anti-PD-1 antibody alone, Anti-PD-1 antibody + Lipoplex group that used the anti-PD-1 antibody and the lipoplex in combination did not show statistically significant difference in the efficacy. Therefore, it can be seen that only the lipoplex having the composition within a specific range can effectively exert the immune anticancer effect.

**Example 7: evaluation of immune anticancer effect of lipoplex *in vivo***

Example 7 evaluates the influence of different formulations of lipoplexes (different types of PEG lipids) on immune anticancer effect. The same method was used as that in Example 5, $2\times10^5$ cells of CT26 cell line were inoculated to the right back subcutaneous of 5 to 8-week-old mice (female BALB/c mice, purchased from Lesco Biotechnology Co., Ltd.). When the average volume of tumors reached 100 to 200 mm$^3$, the administration was carried out according to the following groups: Control group, Anti-PD-1 antibody + Lipoplex group-1, Anti-PD-1 antibody + Lipoplex group-2.

Control group was intravenously injected with normal saline twice a week, and the injection volume was 100 $\mu$L. The anti-mouse PD-1 antibody and the lipoplex of formula C8 were used in combination in the administration of Anti-PD-1 antibody + Lipoplex group-1, among which the anti-PD-1 antibody was intraperitoneally injected twice a week with the dose of 250 $\mu$g and the volume of 100 $\mu$L, and the lipoplex was intravenously injected once a week with the dose of 15 $\mu$g and the volume of 100 $\mu$L. The anti-mouse PD-1 antibody and the lipoplex of formula C14 were used in combination in the administration of Anti-PD-1 antibody + Lipoplex group-2, among which the anti-PD-1 antibody was intraperitoneally injected twice a week with the dose of 250 $\mu$g and the volume of 100 $\mu$L, and the lipoplex was intravenously injected once a week with the dose of 15 $\mu$g and the volume of 100 $\mu$L. The period of drug administration was 14 days. 8 mice in each group were used for the experiment (n=8), and the tumor volume was measured twice a week. The results are shown in FIG. 8. In addition, the tumor growth inhibition value of each group was calculated, and the results are shown in Table 9.

Table 9

| Tumor growth inhibition value (TGI) (day 11 to day 25) (Mean $\pm$ SEM) (n=8) | |
| --- | --- |
| Control group | -- |
| Anti-PD-1 antibody + Lipoplex group-1 (DSPE-PEG-2K) | 98 $\pm$ 7% |
| Anti-PD-1 antibody + Lipoplex group-2 (DMG-PEG-2K) | 93 $\pm$ 5% |

As shown in FIG. 8 and Table 9, the experimental results are shown in the homologous tumor model of the CT26 cell line, Anti-PD-1 antibody + Lipoplex group-1 and Anti-PD-1 antibody + Lipoplex group-2 that used anti-PD-1 antibody and lipoplexes of different formulations (different types of PEG lipids) in combination all have good tumor inhibitory effect (TGI). There was no significant difference in the efficacy of lipoplexes with different formulations using DSPE-PEG-2K and DMG-PEG-2K.

**Example 8: evaluation of immune anticancer effect of lipoplex *in vivo***

Example 8 evaluates the influence of different formulations of lipoplexes (PEGs with different molecular weights) on immune anticancer effect. The same method was used as that in Example 5, $2\times10^5$ cells of CT26 cell line were inoculated to the right back subcutaneous of 5 to 8-week-old mice (female BALB/c mice, purchased from Lesco Biotechnology Co., Ltd.). When the average volume of tumors reached 100 to 200 $mm^3$, the administration was carried out according to the following groups: Control group, Anti-PD-1 antibody + Lipoplex group-1, Anti-PD-1 antibody + Lipoplex group-2.

Control group was intravenously injected with normal saline twice a week, and the injection volume was 100 $\mu$L. The anti-mouse PD-1 antibody and the lipoplex of formula C11 were used in combination in the administration of Anti-PD-1 antibody + Lipoplex group-1, among which the anti-PD-1 antibody was intraperitoneally injected twice a week with the dose of 250 $\mu$g and the volume of 100 $\mu$L, and the lipoplex was intravenously injected once a week with the dose of 15 $\mu$g and the volume of 100 $\mu$L. The anti-mouse PD-1 antibody and the lipoplex of formula C13 were used in combination in the administration of Anti-PD-1 antibody + Lipoplex group-2, among which the anti-PD-1 antibody was intraperitoneally injected twice a week with the dose of 250 $\mu$g and the volume of 100 $\mu$L, and the lipoplex was intravenously injected once a week with the dose of 15 $\mu$g and the volume of 100 $\mu$L. The period of drug administration was 14 days. 8 mice in each group were used for the experiment (n=8), and the tumor volume was measured twice a week. The results are shown in FIG. 9. In addition, the tumor growth inhibition value of each group was calculated, and the results are shown in Table 10.

Table 10

| Tumor growth inhibition value (TGI) (day 11 to day 25) (Mean $\pm$ SEM) (n=8) | |
| --- | --- |
| Control group | -- |
| Anti-PD-1 antibody + Lipoplex group-1 (DSPE-PEG-1K) | 81 $\pm$ 25% |
| Anti-PD-1 antibody + Lipoplex group-2 (DSPE-PEG-10K) | 82 $\pm$ 21% |

As shown in FIG. 9 and Table 10, the experimental results are shown in the homologous tumor model of the CT26 cell line, Anti-PD-1 antibody + Lipoplex group-1 and Anti-PD-1 antibody + Lipoplex group-2 that used anti-PD-1 antibody and lipoplexes of different formulations (PEGs with different molecular weights, i.e. PEG molecules with different lengths) in combination all have good tumor inhibitory effect (TGI). There was no significant difference in the efficacy of lipoplexes with different formulations using DSPE-PEG-1K and DSPE-PEG-10K.

**Example 9: evaluation of immune anticancer effect of lipoplex *in vivo***

Example 9 evaluates the specificity and memory of lipoplexes for tumors. The mouse colorectal cancer cell line CT26 (purchased from ATCC) was cultured in RPMI 1640 medium (purchased from Thermo Fisher Scientific) supplemented with 10% fetal bovine serum (FBS), the murine breast cancer cell line 4T1 (purchased from ATCC) was cultured in RPMI 1640 medium supplemented with 10% fetal bovine serum (FBS), and they were placed in the incubator at 37°C, 5% $CO_2$. Thereafter, $2\times10^5$ cells of CT26 cell line were inoculated to the right back subcutaneous of 5 to 8-week-old mice (female BALB/c mice, purchased from Lesco Biotechnology Co., Ltd.). When the average volume of tumors reached 100 to 200 $mm^3$, the administration was carried out according to the following: Anti-PD-1 antibody + Lipoplex group-1, Anti-PD-1 antibody + Lipoplex group-2.

Anti-PD-1 antibody + Lipoplex-1 and Anti-PD-1 antibody + Lipoplex-2 were two mice that were both administered with the anti-mouse PD-1 antibody and the lipoplex of formula C1 in combination, among which the anti-PD-1 antibody was intraperitoneally injected twice a week with the dose of 250 $\mu$g and the volume of 100 $\mu$L, and the lipoplex was intravenously injected twice a week with the dose of 50 $\mu$g and the volume of 100 $\mu$L. The period of drug administration was 14 days (day 11 to day 25). 2 mice in each group were used for the experiment (n=2), and the tumor volume was measured once or twice a week. In addition, CT26 cells were re-inoculated to the mice reaching complete remission and the normal mice (as Control group, n=5) on day 81, and 4T1 cells were inoculated in the mice reaching complete remission (n=2) on day 117.

As shown in FIG. 10, the experimental results show that Anti-PD-1 antibody + Lipoplex-1 and Anti-PD-1 antibody + Lipoplex-2 that used anti-PD-1 antibody and lipoplex in combination could make the tumor completely remission after administration. In addition, the tumor-free (complete remission of tumor) mice still had no tumor growth after re-inoculation with mouse colorectal cancer cell line CT26 (n=2). In contrast, normal mice (Control group) developed tumors after inoculation with CT26 cell line (n=5). Moreover, when the aforementioned tumor-free (complete remission of tumor) mice were additionally inoculated with a different tumor cell line (mouse breast cancer cell line 4T1), tumors could grow (n=2). It can be known that the combined use of anti-PD-1 antibody and lipoplex has specific immune memory for the same type of tumor, which can effectively reduce the probability of tumor recurrence in clinical application.

**Example 10: evaluation of TLR9 activation ability of lipoplex**

Example 10 evaluates the influence of lipoplexes loaded with different types of CpG oligodeoxynucleotides (CpG-ODNs) on the ability to activate TLR9. The experimental method of Example 10 was substantially similar to that of Example 2. However, in Example 10, the HEK-Blue™ mTLR9 cell line (purchased from InvivoGen, product number hkb-mtlr9) expressing mouse TLR9 was used to evaluate the TLR9 activation ability of the lipoplex of formula C20 (Lipoplex group) and CpG-30-PS alone (CpG-ODN group).

As shown in FIG. 11, the experimental results show that the $EC_{50}$ value of CpG-ODN group was about 419 nM, and the $EC_{50}$ value of Lipoplex group was about 9 nM. Compared with CpG oligodeoxynucleotide used alone, the lipoplexes loaded with different types of CpG oligodeoxynucleotides can also greatly enhance the activation ability of TLR9 ($EC_{50}$ value increased by about 45 times).

**Example 11: evaluation of cytokine activation ability of lipoplex**

Example 11 evaluates the influence of lipoplexes loaded with different types of CpG oligodeoxynucleotides (CpG-ODNs) on the ability to activate TLR9. The experimental method of Example 11 was substantially similar to that of Example 4. However, the samples used in Example 11 were CpG-30-PS (CpG-ODN group) and the lipoplex of formula C20 (Lipoplex group).

As shown in FIGs. 12A to 12B, the experimental results show that Lipoplex group could promote the production of IL-6 and IFN-γ in human PBMC cells at a low concentration (for example, about 10 nM), and the activation ability was higher than that of using CpG -30-PS alone (CpG-ODN group). Therefore, the lipoplexes carrying different types of CpG oligodeoxynucleotides can also effectively promote the secretion of cytokines related to immune and anti-cancer.

**Example 12: safety assessment of lipoplex**

Example 12 evaluates the influence of the contents of PEG lipids and immunostimulatory nucleic acid drugs in lipoplex on formula safety. Repeated dose toxicity test was carried out with BALB/c mice (purchased from Lesco Biotechnology Co., Ltd.), which was administered intravenously once a week, and the period of administration was 14 days. The mice were weighed and clinically observed during the test. The administration of the experiments was carried out according to the following groups: Blank control group, Lipoplex group-1, Lipoplex group-2, Lipoplex group-3, and Lipoplex group-4.

Control group was intravenously injected with normal saline once a week, and the injection volume was 100 μL. Lipoplex group-1 was injected with the lipoplex of formula C15, the injection dose was 50 μg, and the injection volume was 100 μL. Lipoplex group-2 was injected with the lipoplex of formula C2, the injection dose was 50 μg, and the injection volume was 100 μL. Lipoplex group-3 was injected with the lipoplex of formula C9, the injection dose was 15 μg, and the injection volume was 100 μL. Lipoplex group-4 was injected with the lipoplex of formula C9, the injection dose was 5 μg, and the injection volume was 100 μL. The period of administration was 11 days, and the experiment was performed with 5 mice in each group (n=5). The results are shown in FIGs. 13A to 13B.

As shown in FIGs. 13A to 13B, Lipoplex group-1 lost about 14% of body weight on the second day of administration, and two mice died. The rest of the mice were sacrificed for necropsy sampling, and adverse reactions such as lung parenchyma, pleural effusion, obvious liver lobular septa, and no eating of some mice were found. Thus, it can be seen that the content of PEG lipids in the lipoplex formulation has a great influence on the safety. Therefore, the composition of lipoplexes should contain PEG lipids.

As described above, the novel nucleic acid-drug complex provided in the embodiments of the present disclosure combines CpG oligonucleotide sequence and anti-PD-L1 aptamer, which has tumor targetability and immune checkpoint-blocking activity, and can increase the accumulation of nucleic acid-drug complexes in tumors and the immune cytotoxicity in tumor microenvironment. In addition, the nucleic acid-drug complex has the ability to stimulate the activation of a variety of immune cells, and can increase the activation and aggregation of immune cells in tumor microenvironment.

Moreover, the nucleic acid-drug complexes provided in the embodiments of the present disclosure have better anti-tumor efficacy than simply combining the uses of CpG oligonucleotides and anti-PD-Ll aptamers.

As described above, the immunostimulatory lipoplex provided in the embodiments of the present disclosure includes the liposome with a specific composition and the immunostimulatory nucleic acid drug that is complexed with the liposome. The immunostimulatory lipoplex can be administered using a systemic administration manner. The lipoplex can improve the problems of poor stability and insufficient tissue exposure of immunostimulatory nucleic acid drugs. The specific immune activation of the drugs therefore can be improved, and the action time of a single dose of drugs can be prolonged, which reduces the systemic immune side effects caused by the drugs. Furthermore, in accordance with some embodiments of the present disclosure, the combined use of the immunostimulatory lipoplex and the current immune checkpoint inhibitor can exert a synergistic effect to further improve the efficacy of cancer immunotherapy.

Although some embodiments of the present disclosure and their advantages have been described as above, it should be understood that various changes, substitutions and alterations can be made herein without departing from the spirit and scope of the disclosure as defined by the appended claims. In addition, each claim constitutes an individual embodiment, and the claimed scope of the present disclosure also includes the combinations of the claims and embodiments. The scope of protection of the present disclosure is subject to the definition of the scope of the appended claims.

## Claims

1. An immunostimulatory lipoplex, comprising:

   a liposome; and
   at least one immunostimulatory nucleic acid drug complexed with the liposome;
   wherein the liposome comprises 40 to 85 mole percent of cationic lipid, 10 to 50 mole percent of cholesterol,
   and 0.001 to 20 mole percent of modified polyethylene glycol (PEG) lipid.

2. The immunostimulatory lipoplex as claimed in claim 1, wherein the immunostimulatory nucleic acid drug comprises
   CpG oligodeoxynucleotide (CpG-ODN), small interfering RNA (siRNA), microRNA (miRNA), or a combination thereof.

3. The immunostimulatory lipoplex as claimed in claim 1 or 2, wherein the cationic lipid comprises 1,2-dioleoyl-3-trimethylammonium propane (DOTAP), 1,2-di-O-octadecenyl-3-trimethylammonium propane (DOTMA), didodecyldimethylammonium bromide (DDAB), 1,2-dioleyloxy-3-dimethylamino propane (DODMA), lipid GL67 (Genzyme Lipid 67), ethyl phosphocholine (ethyl PC), $3\beta$-[N-(N',N'-dimethylaminoethane)-carbamoyl]cholesterol (DC-cholesterol), derivatives of the foregoing cationic lipid, or a combination of the foregoing cationic lipids and derivatives thereof.

4. The immunostimulatory lipoplex as claimed in any one of claims 1 to 3, wherein the modified PEG lipid comprises DSPE-PEG lipid, DMG-PEG lipid, or a combination thereof.

5. The immunostimulatory lipoplex as claimed in claim 4, wherein a PEG average molecular weight of the DSPE-PEG lipid and the DMG-PEG lipid each is in a range from 500 to 15000 Da.

6. The immunostimulatory lipoplex as claimed in claim 4, wherein a PEG terminal functional group of the DSPE-PEG lipid and the DMG-PEG lipid comprises amine group ($NH_2$) or maleimide group.

7. The immunostimulatory lipoplex as claimed in any one of claims 1 to 6, wherein a particle diameter of the immunostimulatory lipoplex is in a range from 50 nanometers to 350 nanometers, and a polymer dispersity index (PDI) of the immunostimulatory lipoplex is less than 0.4.

8. A use of the immunostimulatory lipoplex as claimed in any one of claims 1 to 7, which is used for the manufacture of a medicine for treating cancer.

9. The use of the immunostimulatory lipoplex as claimed in claim 8, wherein the cancer comprises colon cancer, breast cancer, lung cancer, pancreatic cancer, liver cancer, stomach cancer, esophageal cancer, head and neck squamous cell carcinoma, prostate cancer, bladder cancer, lymphoma, gallbladder cancer, kidney cancer, blood cancer, colorectal cancer, multiple myeloma, ovarian cancer, cervical cancer or glioma.

10. The use of the immunostimulatory lipoplex as claimed in claim 8, wherein a mode of administration of the immunostimulatory lipoplex to an individual comprises intravenous injection, subcutaneous injection, intramuscular injection, or inhalation.

11. The use of the immunostimulatory lipoplex as claimed in any one of claims 8 to 10, wherein the immunostimulatory lipoplex is used in combination with an immune checkpoint inhibitor, and the immune checkpoint inhibitor comprises anti-PD-1/PD-L1 antibody, anti-CTLA-4 antibody, or a combination thereof.

12. A pharmaceutical composition, comprising an immunostimulatory lipoplex as claimed in any one of claims 1 to 7.

13. The pharmaceutical composition as claimed in claim 12, further comprising an immune checkpoint inhibitor, which is used in combination with the immunostimulatory lipoplex, wherein the immune checkpoint inhibitor comprises anti-PD-1/PD-L1 antibody, anti-CTLA-4 antibody, or a combination thereof.

14. A use of the pharmaceutical composition as claimed in claim 12 or 13, which is used for the manufacture of a medicine for treating cancer.

15. A kit, comprising the pharmaceutical composition as claimed in claim 12 or 13.

Mouse tissue distribution

FIG. 1

FIG. 2

FIG. 3

FIG. 4A

FIG. 4B

FIG. 4C

EP 4 019 006 A1

FIG. 5

FIG. 6

FIG. 7

FIG. 8

FIG. 9

FIG. 10

FIG. 11

IL-6

FIG. 12A

IFN-γ

FIG. 12B

FIG. 13A

FIG. 13B

EP 4 019 006 A1

Europäisches
Patentamt

European
Patent Office

Office européen
des brevets

## EUROPEAN SEARCH REPORT

**Application Number**

EP 21 21 7405

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/263407 A1 (DANDE PRASAD A [US] ET AL) 22 October 2009 (2009-10-22) * paragraphs [0004], [0284], [0318], [0333]; tables 2, 4-5 * | 1-15 | INV. A61K9/127 |
| X | WO 2009/127060 A1 (PROTIVA BIOTHERAPEUTICS INC [CA]; MACLACHLAN IAN [CA] ET AL.) 22 October 2009 (2009-10-22) * paragraphs [0071], [0256], [0286], [0339], [0327]; claims 1, 38, 43-46 * | 1-15 | |
| X | MIRKO TRAJKOVSKI ET AL: "MicroRNAs 103 and 107 regulate insulin sensitivity", NATURE, vol. 474, no. 7353, 30 June 2011 (2011-06-30), pages 649-653, XP055222703, London ISSN: 0028-0836, DOI: 10.1038/nature10112 * page 5, left-hand column, last paragraph - right-hand column, paragraph 1st * | 1-7 | |
| X | SCHEIDELER MARCEL ET AL: "Lipid nanocarriers for microRNA delivery", CHEMISTRY AND PHYSICS OF LIPIDS, LIMERICK, IR, vol. 226, 2 November 2019 (2019-11-02), XP085975068, ISSN: 0009-3084, DOI: 10.1016/J.CHEMPHYSLIP.2019.104837 [retrieved on 2019-11-02] * page 9, left-hand column, paragraph 3; table 1 * | 1-10,12, 14,15 | **TECHNICAL FIELDS SEARCHED (IPC)** A61K |

The present search report has been drawn up for all claims

| Place of search | Date of completion of the search | Examiner |
|---|---|---|
| The Hague | 10 May 2022 | Schwald, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 7405

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2022

| Patent document cited in search report | | Publication date | Patent family member(s) | | Publication date |
|---|---|---|---|---|---|
| US 2009263407 | A1 | 22-10-2009 | NONE | | |
| WO 2009127060 | A1 | 22-10-2009 | AU | 2009238175 A1 | 22-10-2009 |
| | | | CA | 2721333 A1 | 22-10-2009 |
| | | | CN | 102119217 A | 06-07-2011 |
| | | | DK | 2279254 T3 | 18-09-2017 |
| | | | EP | 2279254 A1 | 02-02-2011 |
| | | | ES | 2638448 T3 | 20-10-2017 |
| | | | HU | E034483 T2 | 28-02-2018 |
| | | | IL | 208744 A | 29-12-2016 |
| | | | JP | 5475753 B2 | 16-04-2014 |
| | | | JP | 2011516586 A | 26-05-2011 |
| | | | NZ | 588583 A | 31-08-2012 |
| | | | PL | 2279254 T3 | 30-11-2017 |
| | | | PT | 2279254 T | 04-09-2017 |
| | | | US | 2010130588 A1 | 27-05-2010 |
| | | | US | 2012183581 A1 | 19-07-2012 |
| | | | US | 2014065228 A1 | 06-03-2014 |
| | | | US | 2015164799 A1 | 18-06-2015 |
| | | | US | 2017042814 A1 | 16-02-2017 |
| | | | US | 2018085312 A1 | 29-03-2018 |
| | | | US | 2018092848 A1 | 05-04-2018 |
| | | | US | 2020113832 A1 | 16-04-2020 |
| | | | US | 2021220274 A1 | 22-07-2021 |
| | | | US | 2021267895 A1 | 02-09-2021 |
| | | | WO | 2009127060 A1 | 22-10-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63131134

**Non-patent literature cited in the description**

- *CHEMICAL ABSTRACTS,* 132172-61-3
- *CHEMICAL ABSTRACTS,* 57-88-5
- *CHEMICAL ABSTRACTS,* 247925-28-6
- *CHEMICAL ABSTRACTS,* 474922-26-4
- *CHEMICAL ABSTRACTS,* 474922-22-0